(19) **Europäisches Patentamt** / **European Patent Office** / **Office européen des brevets**

(11) **EP 1 799 852 B1**

(12) **EUROPEAN PATENT SPECIFICATION**

(45) Date of publication and mention
of the grant of the patent:
**08.07.2009 Bulletin 2009/28**

(21) Application number: **05783528.2**

(22) Date of filing: **20.09.2005**

(51) Int Cl.:
***C12Q 1/68*** (2006.01)

(86) International application number:
**PCT/IS2005/000023**

(87) International publication number:
**WO 2006/048896 (11.05.2006 Gazette 2006/19)**

(54) **ASSOCIATION OF GENE EXPRESSION AND PSYCHIATRIC DISORDERS**

ZUSAMMENHANG VON GENEXPRESSION UND PSYCHIATRISCHEN STÖRUNGEN

ASSOCIATION D'EXPRESSION GENETIQUE ET DE TROUBLES PSYCHIATRIQUES

(84) Designated Contracting States:
**AT BE BG CH CY CZ DE DK EE ES FI FR GB GR HU IE IS IT LI LT LU LV MC NL PL PT RO SE SI SK TR**

(30) Priority: **20.09.2004 US 611401 P**

(43) Date of publication of application:
**27.06.2007 Bulletin 2007/26**

(73) Proprietor: **Decode Genetics EHF**
**101 Reykjavik (IS)**

(72) Inventors:
• **THORLEIFSSON, Gudmar**
**111 Reykjavik (IS)**
• **KONG, Augustine**
**Chicago, Illinois 60637 (US)**
• **THORGEIRSSON, Thorgeir E.**
**IS-107 Reykjavik (IS)**

(74) Representative: **Arnason Faktor**
**Intellectual Property Consulting**
**Gudridarstig 2-4**
**IS-113 Reykjavik (IS)**

(56) References cited:
**WO-A-00/05407** **WO-A-20/05040427**

• **THORGEIRSSON T E ET AL: "Markers associated with the inversion polymorphism on chromosome 8p23 are associated with Panic Disorders" AMERICAN JOURNAL OF HUMAN GENETICS, AMERICAN SOCIETY OF HUMAN GENETICS, CHICAGO, IL, US, vol. 73, no. 5, November 2003 (2003-11), page 514, XP002343054 ISSN: 0002-9297**

• **GIGLIO S ET AL: "Olfactory receptor-gene clusters, genomic-inversion polymorphisms, and common chromosome rearrangements." AMERICAN JOURNAL OF HUMAN GENETICS. APR 2001, vol. 68, no. 4, April 2001 (2001-04), pages 874-883, XP007900195 ISSN: 0002-9297 cited in the application**

• **SHIMOKAWA OSAMU ET AL: "Molecular characterization of inv dup del(8p): analysis of five cases." AMERICAN JOURNAL OF MEDICAL GENETICS. PART A. 15 JUL 2004, vol. 128, no. 2, 15 July 2004 (2004-07-15), pages 133-137, XP007900212 ISSN: 1552-4825**

• **SUGAWARA H ET AL: "Complex low-copy repeats associated with a common polymorphic inversion at human chromosome 8p23" GENOMICS, ACADEMIC PRESS, SAN DIEGO, US, vol. 82, no. 2, August 2003 (2003-08), pages 238-244, XP004434191 ISSN: 0888-7543**

• **MACINTYRE D J ET AL: "Chromosomal abnormalities and mental illness" MOLECULAR PSYCHIATRY, BASINGSTOKE, GB, vol. 8, no. 3, March 2003 (2003-03), pages 275-287, XP002343058 ISSN: 1359-4184**

• **OPHOFF R A ET AL: "Genomewide linkage disequilibrium mapping of severe bipolar disorder in a population isolate" AMERICAN JOURNAL OF HUMAN GENETICS, AMERICAN SOCIETY OF HUMAN GENETICS, CHICAGO, IL, US, vol. 71, no. 3, September 2002 (2002-09), pages 565-574, XP002343055 ISSN: 0002-9297**

• **PULVER A E: "Search for schizophrenia susceptibility genes" BIOLOGICAL PSYCHIATRY, ELSEVIER SCIENCE, NEW YORK, NY, US, vol. 47, 1 February 2000 (2000-02-01), pages 221-230, XP000944356 ISSN: 0006-3223**

EP 1 799 852 B1

**Description**

BACKGROUND OF THE INVENTION

In general terms, panic disorder is a manifestation of anxiety in which feelings of extreme fear and dread strike unexpectedly and repeatedly for no apparent reason, accompanied by intense physical symptoms. Panic disorder is characterized by unexpected and repeated episodes of intense fear accompanied by physical symptoms that can include chest pain, heart palpitations, shortness of breath, dizziness or abdominal distress. About 1.7% of the adult U.S. population ages 18 to 54 - approximately 2.4 million Americans - has panic disorder in a given year. Panic disorder affects about 1 out of 75 people worldwide. Women are twice as likely as men to develop panic disorder. Panic disorder typically strikes in young adulthood. Roughly half of all people who have panic disorder develop the condition before age 24.

Many people with panic disorder develop intense anxiety between episodes. It is not unusual for a person with panic disorder to develop phobias about places or situations where panic attacks have occurred, such as in supermarkets or other everyday situations. As the frequency of panic attacks increases, the person often begins to avoid situations where they fear another attack may occur or where help would not be immediately available. This avoidance can develop into agoraphobia, an inability to go beyond known and safe surroundings because of intense fear and anxiety.

Panic disorder can coexist with other comorbid disorders, *e.g.*, depression, bipolar disorder (also known as manic-depressive illness; a brain disorder that causes unusual shifts in a person's mood, energy, and ability to function), obsessive-compulsive disorder (characterized by intrusive, unwanted, repetitive thoughts and rituals performed out of a feeling of urgent need), histrionic personality disorder, family denial and dysfunction, hypercholesterolemia and substance abuse. About 30% of people with panic disorder abuse alcohol and 17% abuse drugs, such as cocaine and marijuana, in unsuccessful attempts to alleviate the anguish and distress caused by their condition. Appropriate diagnosis and treatment of other disorders such as, for example, depression, bipolar disorder and substance abuse, are important to successfully treat panic disorder.

Heredity, other biological factors, stressful life events, and thinking in a way that exaggerates relatively normal bodily reactions are all believed to play a role in the onset of panic disorder. The exact cause or causes of panic disorder are unknown and are the subject of intense scientific investigation.

Studies in animals and humans have focused on pinpointing the specific brain areas and circuits involved in anxiety and fear, which underlie anxiety disorders such as panic disorder. Fear, an emotion that evolved to deal with danger, causes an automatic, rapid protective response that occurs without the need for conscious thought. It has been found that the body's fear response is coordinated by a small structure deep inside the brain, called the amygdala. The amygdala, although relatively small, is a very complicated structure, and recent research suggests that anxiety disorders are associated with abnormal activity in the amygdala.

Treatment for panic disorder can consist of taking a medication to adjust the chemicals in the body, or treatment might involve working with a psychotherapist to gain more control over your anxieties. Both types of treatment can be very effective. For many patients, the combination of medication and psychotherapy appears to be more effective than either treatment alone. Early treatment can help keep panic disorder from progressing. Therefore, early diagnosis of panic disorder is essential for providing effective treatment. The symptoms associated with panic disorder (*e.g.*, chest pain, heart palpitations, shortness of breath, dizziness or abdominal distress) often mimic symptoms of a heart attack or other life-threatening medical conditions. As a result, the diagnosis of panic disorder is frequently not made until extensive and costly medical procedures, fail to provide a correct diagnosis or relief.

Depressive Illness is among the most common and destructive of illnesses prevalent in the United States today and according to WHO statistics; major depression is the leading cause of disability worldwide (Murray C., Lopez A., eds. The global burden of disease: a comprehensive assessment of mortality and disability from diseases, injuries, and risk factors in 1990 and projected to 2020. Harvard University Press, 1996. Cambridge, MA). Depressive disorders affect an estimated 9.5 percent of adult Americans ages 18 and over in a given year, or about 18.8 million people in 1998. An estimated 35 - 40 million Americans living today will suffer from major depressive illness during their lives. For each person directly suffering, three or four times that number of their relatives, employees, associates, and friends will also be adversely affected due to their relationship with the person directly suffering. Of those 35 - 40 million afflicted, a substantial percentage will commit suicide if not treated with appropriate medication (*e.g.*, amine reuptake inhibitors, selective serotonin reuptake inhibitors, selective norepinephrine reuptake inhibitors, combined serotonin-norepinephrine reuptake inhibitors, combined dopamine-norepinephrine reuptake inhibitors, monoamine oxidase inhibitors tricyclic drugs).

Standard criteria for depression include an abnormal sense of sadness and despair, disordered eating and weight control, diminished sexual interest and abnormal sleeping patterns. Furthermore, depression can be classified as exogenous or endogenous, major or minor, and unipolar or dipolar depending on its time course, severity, and cyclicity (if present). In addition to major depression, many people suffer from manic-depressive illness (bipolar disorder; BPD) that is characterized by radical mood swings from severe depression to exaggerated, inappropriate elation. Evidence from twin studies suggests that many depressive illnesses demonstrate a genetic disposition although a precise etiology remains undefined. However, all major theories of depression address neurophysiological mechanisms as part of the cause of depressive illness.

WO 00/05407 A (2000-02-03) discloses a method for diagnosing a psychiatric disorder comprising the detection of the over-expression, preferably in form of duplication of the genomic sequence of the human chromosome 15q24-25.

SUMMARY

The present invention is based upon the discovery of the relationship between the orientation of the Inv8p23 polymorphism and gene expression. The orientation of the Inv8p23 inversion fragment is linked to psychiatric disorders, *e.g.*, anxiety disorder such as, for example, panic disorder, bipolar disorder and depression (see PCT application WO05040427A2, "Inversion on Chromosome 8p23 is Risk Factor For Anxiety Disorders, Depression and Bipolar Disorders."). In a first aspect, a method for diagnosing a psychiatric disorder or a comorbid disorder in an individual is described, the method comprising: a) obtaining a gene expression profile from a sample obtained from an individual to be diagnosed; and b) comparing the gene expression profile of the sample with a reference gene expression profile, wherein the reference profile is representative of a specific orientation of the Inv8p23 genomic region, and wherein similarity between the sample expression profile and the reference expression profile is indicative of a psychiatric disorder.

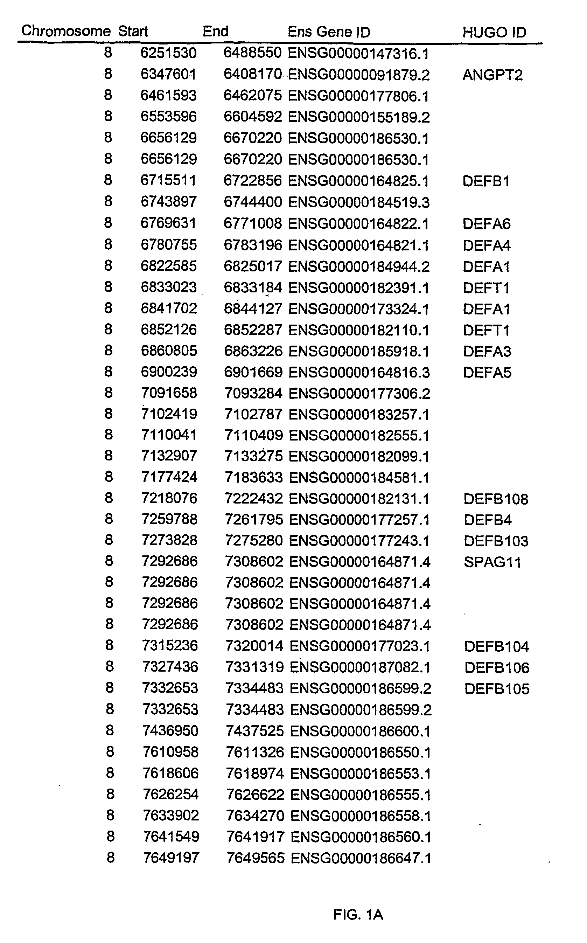

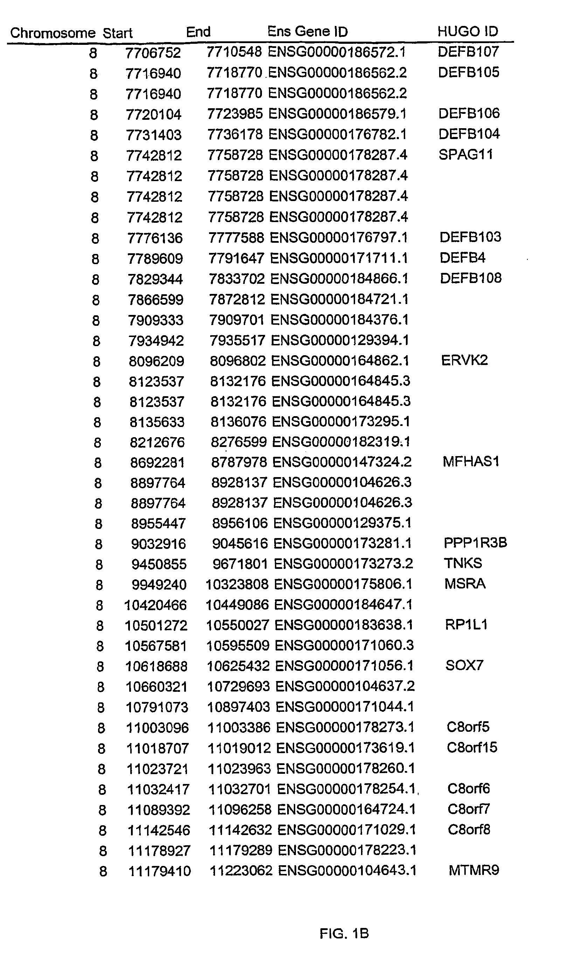

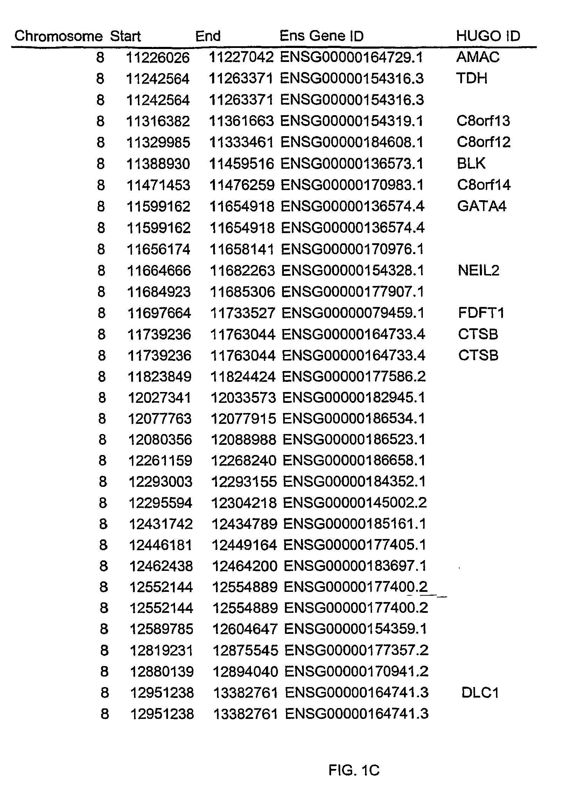

In a particular embodiment, the psychiatric disorder is an anxiety disorder. In a more particular embodiment, the anxiety disorder is panic disorder or bipolar disorder. In another embodiment, the expression profile is indicative of the inverted orientation of the Inv8p23 genomic region. In one embodiment, the comorbid disorder is selected from the group consisting of: depression, bipolar disorder, obsessive-compulsive disorder, histrionic personality disorder, family denial and dysfunction, hypercholesterolemia and substance abuse. In a particular embodiment, the comorbid disorder is selected from the group consisting of: depression, bipolar disorder and hypercholesterolemia. In one embodiment, the gene expression profile comprises the gene expression profile of one or more genes selected from the group consisting of those genes listed in FIGS. 1A-1C. In a particular embodiment, the gene expression profile comprises the gene expression profile of one or more genes selected from the group consisting of: NEIL2, BLK, MSRA, C80RF13, PPP1R3B, TNKS, CTSB, ANGPT, TDH and C80RF7. In another embodiment, the gene expression profile of the sample is obtained using a hybridization assay to oligonucleotides contained in a microarray. In yet another embodiment, the gene expression profile of the sample is obtained by detecting the protein products of the informative genes. In a particular embodiment, antibodies capable of specifically binding protein products of the informative genes are used to detect the protein products of the informative genes.

In another embodiment, the orientation of the Inv8p23 genomic region is the inverted orientation.

In a second aspect, a method of predicting the efficacy of drug treatment of a psychiatric disorder or a comorbid disorder in an individual is described, the method comprising: a) obtaining a gene expression profile from a sample obtained from the individual to be treated; and
b) comparing the gene expression profile of the sample with a reference gene expression profile, wherein the reference profile is representative of a specific orientation of the Inv8p23 genomic region, and wherein similarity between the sample expression profile and the reference expression profile is indicative of a the efficacy of the drug treatment. In one preferred embodiment the psychiatric disorder is an anxiety disorder. In another preferred embodiment, the anxiety disorder is panic disorder or bipolar disorder.

BRIEF DESCRIPTION OF THE DRAWINGS

FIGS. 1A-1C show a partial listing of genes that have been analyzed and described herein or are suitable for expression analysis. This list is not exhaustive, but representative of possible genes for analysis. FIGS. 1A-1C show the existing probes at Applied Biosystems, Foster City, CA.

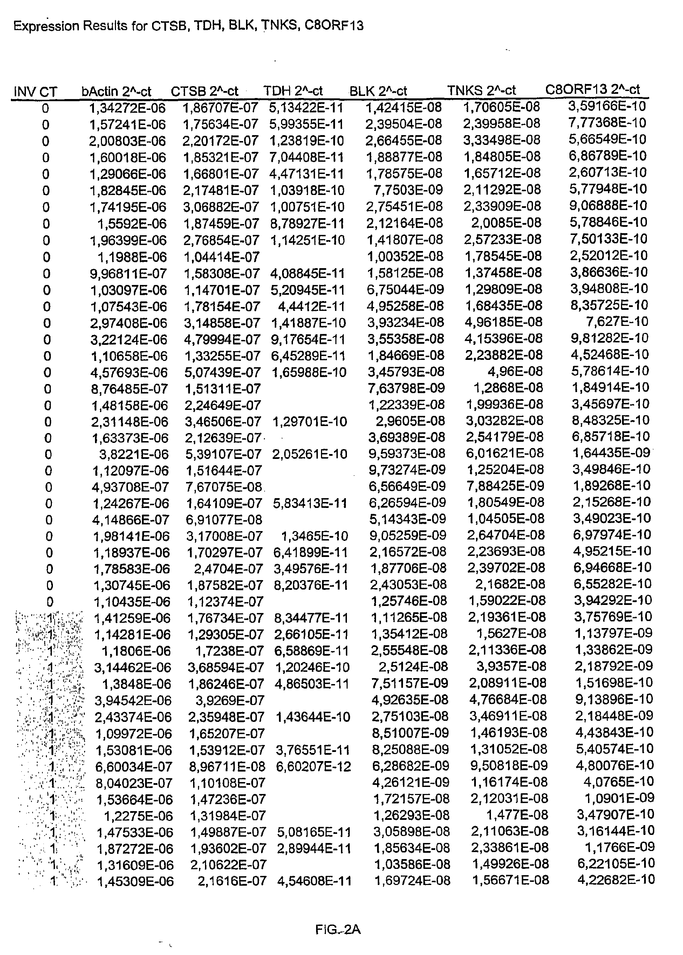

FIGS. 2A-2H show the expression data for the genes CTSB, TDH, BLK, TNKS, C8ORF13 and the results of analyses comparing separately for each of the genes, the mean expression values for the group with an inversion count of 2 to those for the group with an inversion count of 0. Pearson correlation coefficients and the results of t-tests assuming equal variances are shown.

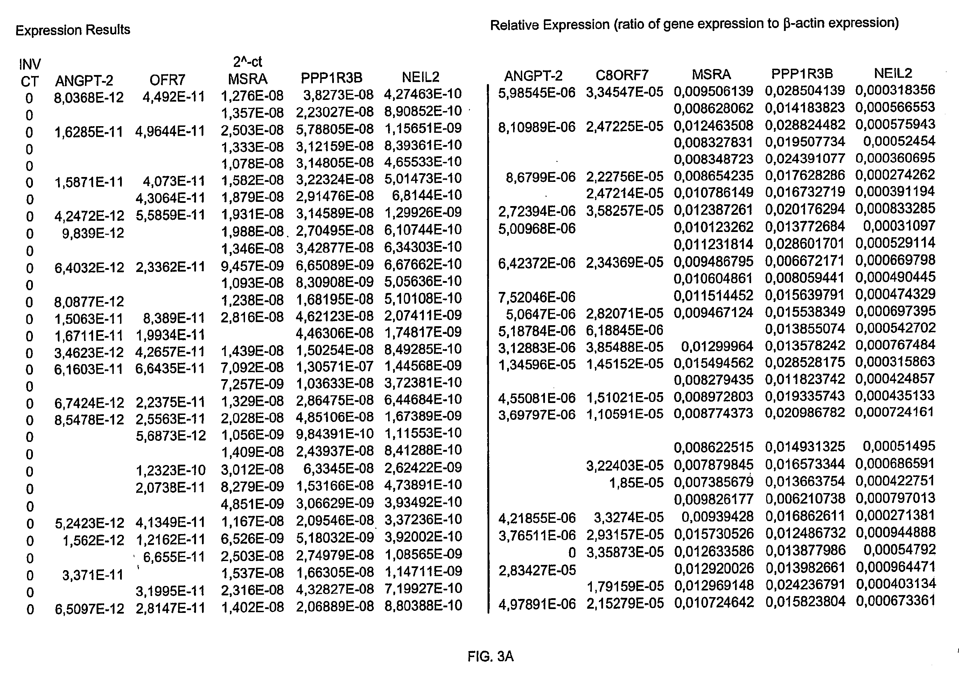

FIGS. 3A-3E show the expression data for the genes ANGPT2, C8ORF7, MSRA, PPP1R3B, NEIL2, and also the results of analyses comparing, separately for each of the genes, the mean expression values for the group with an inversion count of 2 to those for the group with an inversion count of 0. Pearson correlation coefficients and the results of t-tests assuming equal variances are shown.

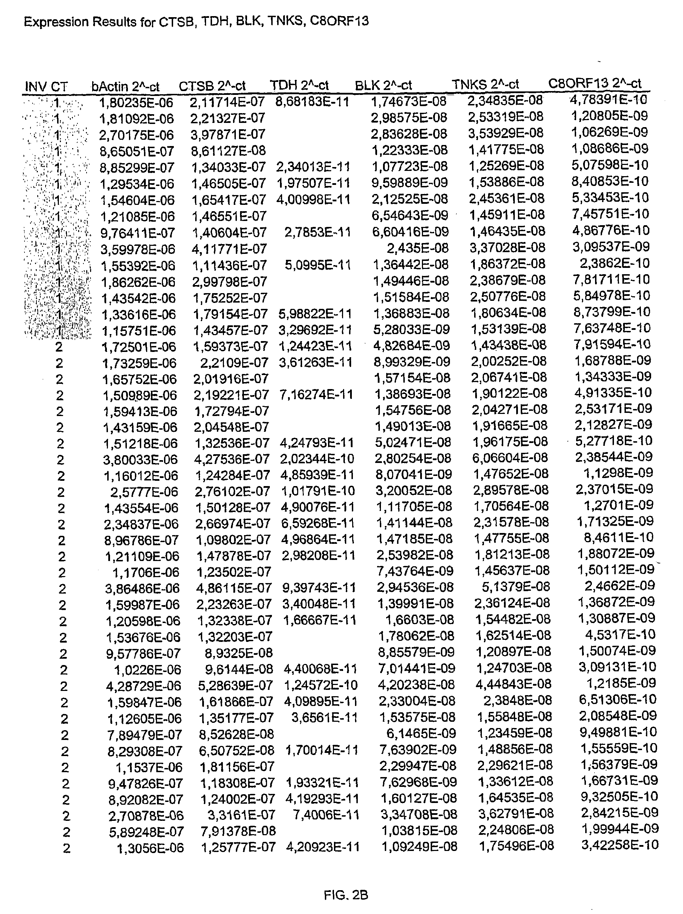

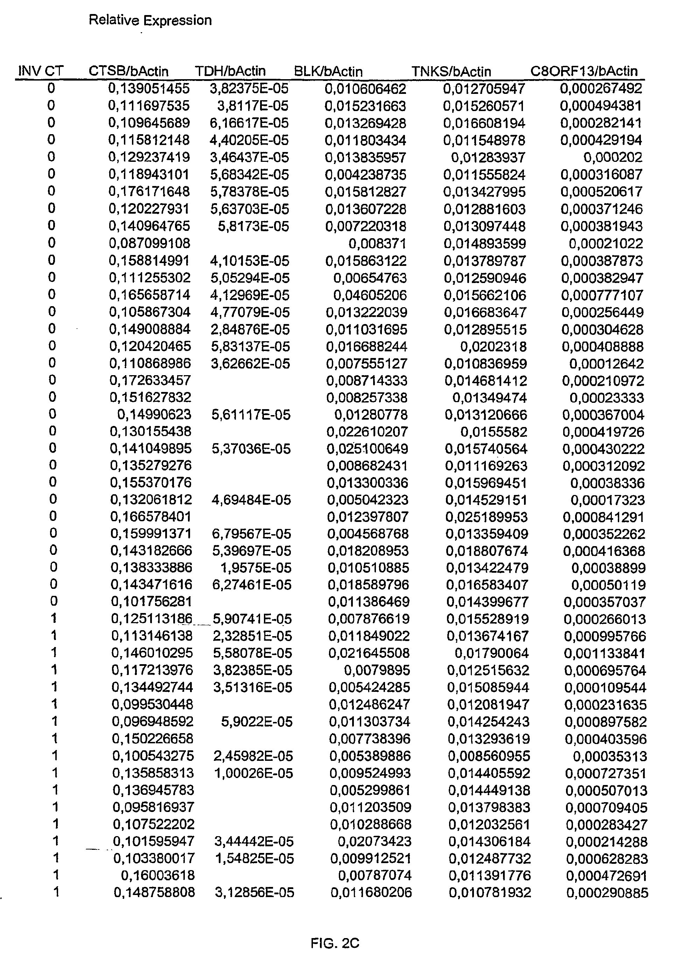

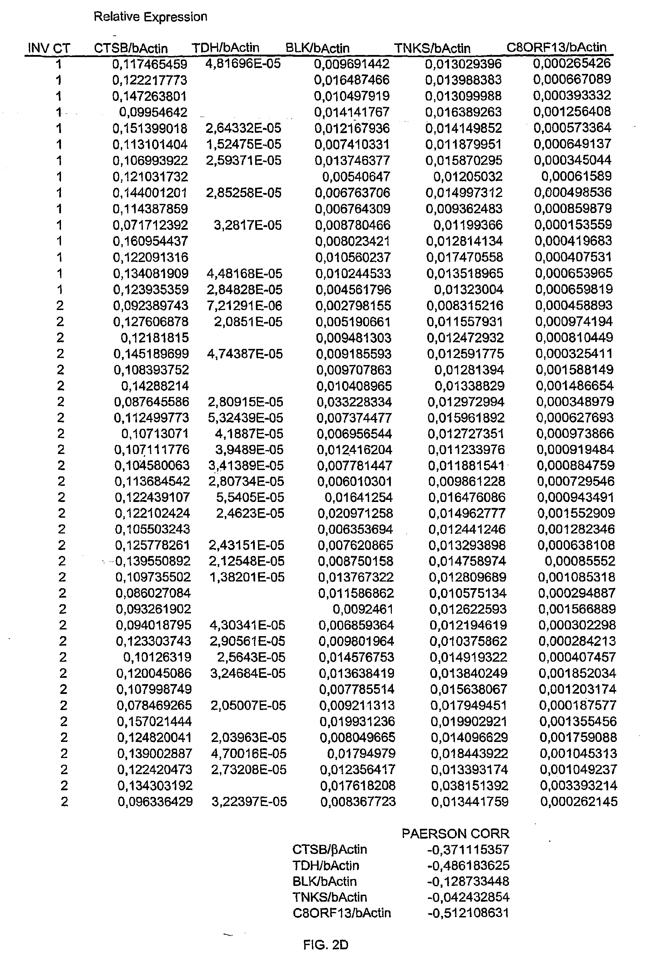

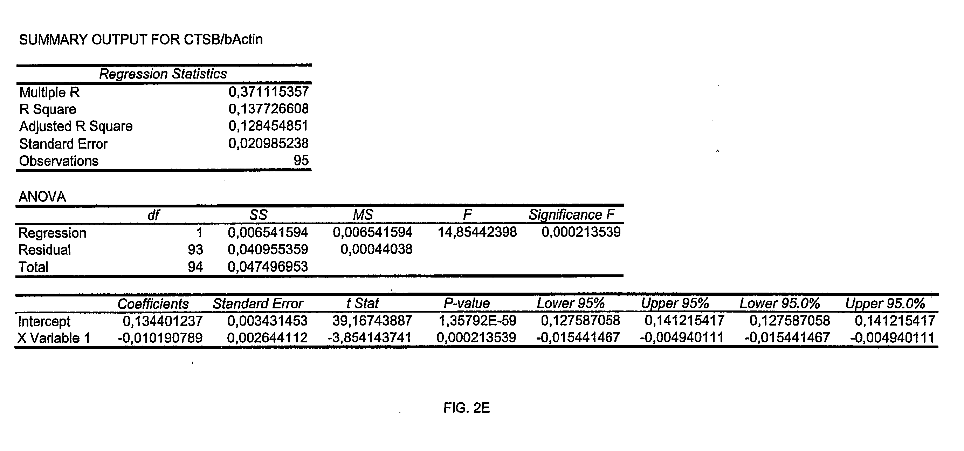

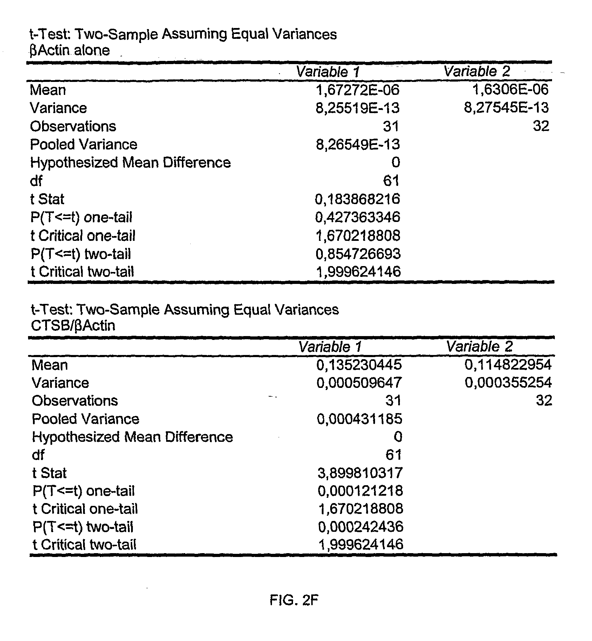

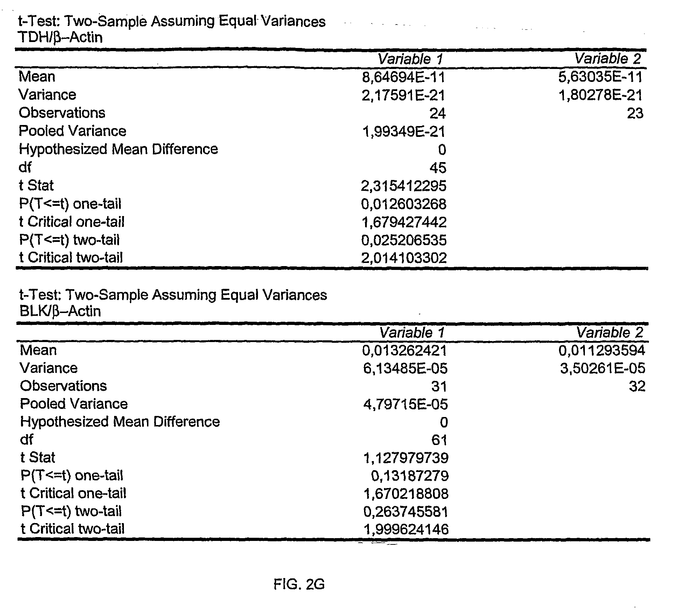

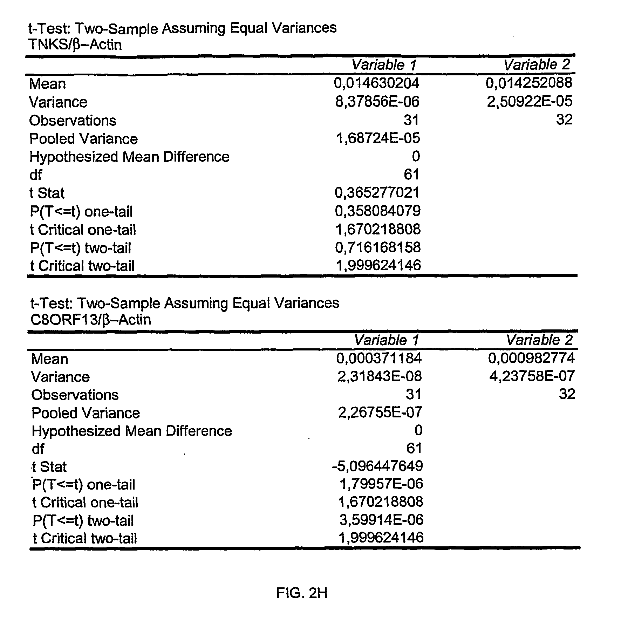

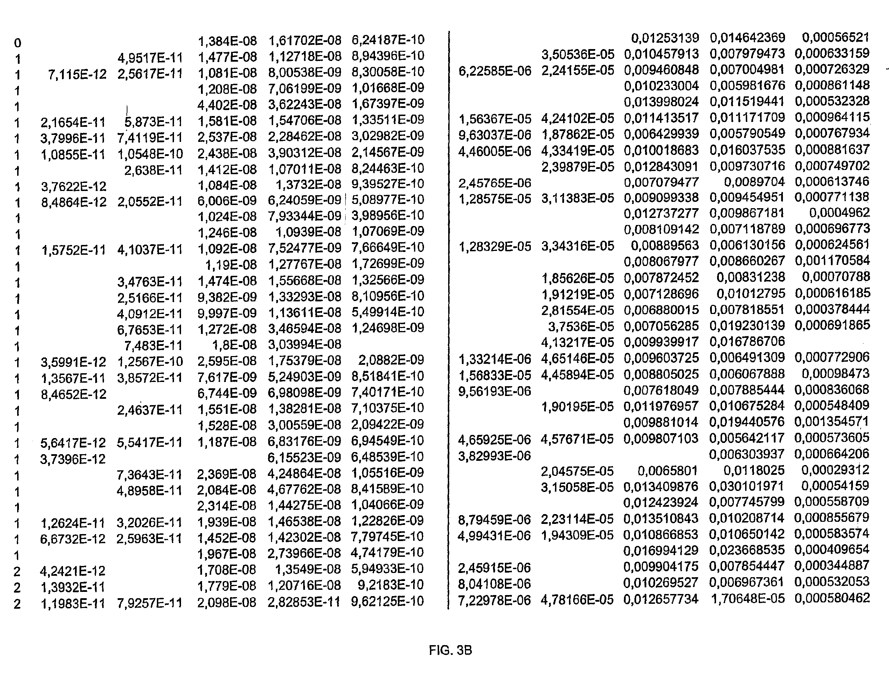

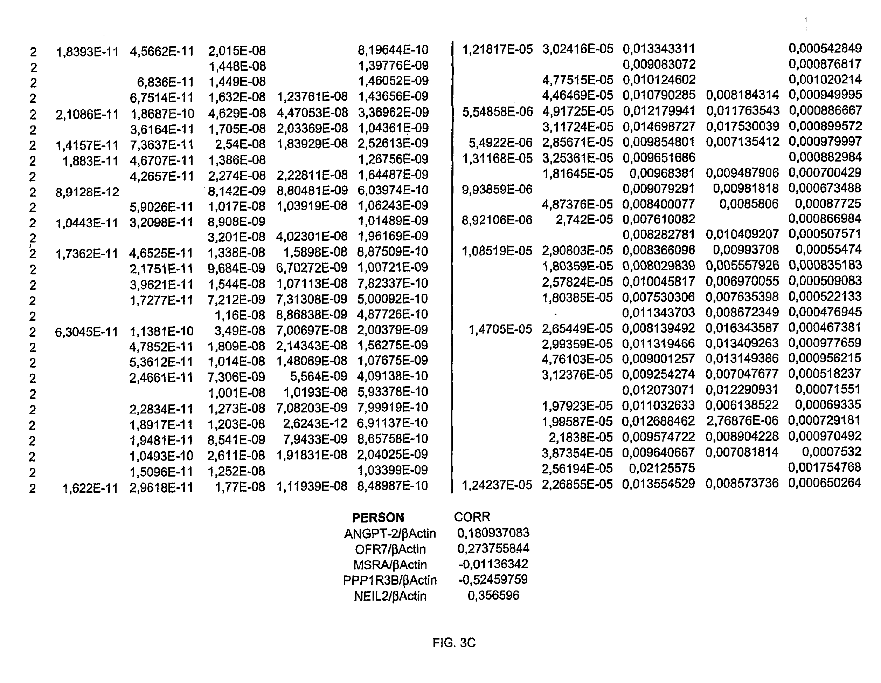

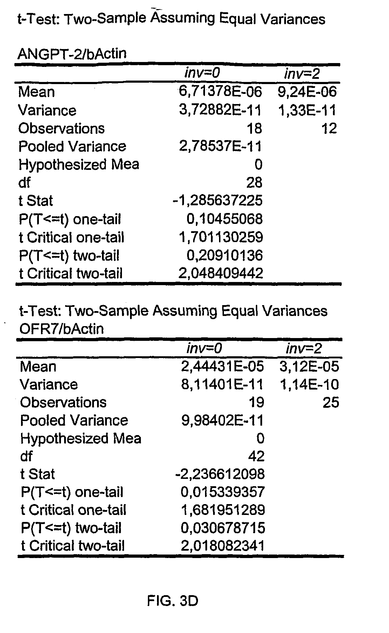

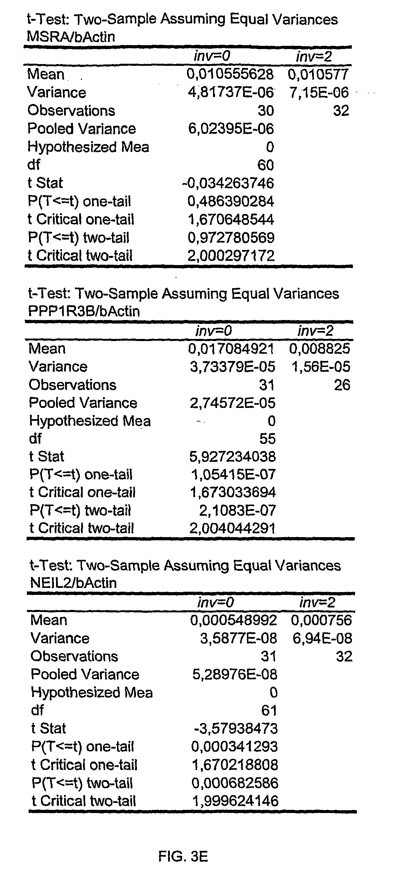

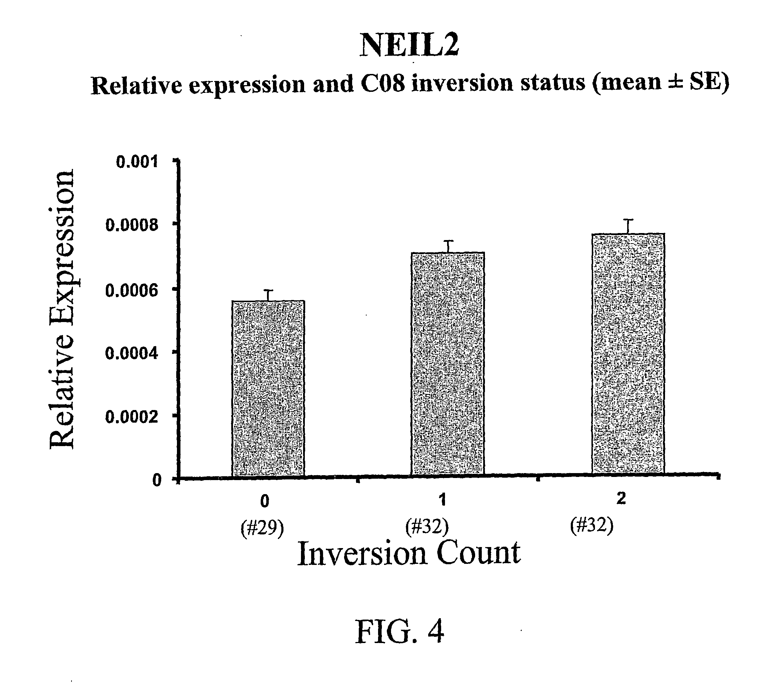

FIG. 4 shows a Bar-graph of the mean expression values for NEIL2 (from FIGS. 2 and 3) for the 3 groups (with inversion counts of 0, 1, and 2; see Example 1). Each group is represented by a bar, and at the top of the bar the standard error is indicated. Also depicted are the results of association analyses where natural logarithm of expressions relative to β-actin on the inversion count. Regressing log-transformed relative expression on estimated number of inverted chromosomes gives : P-value = 0.0004 and correlation coefficient $R^2$ = 0.127.

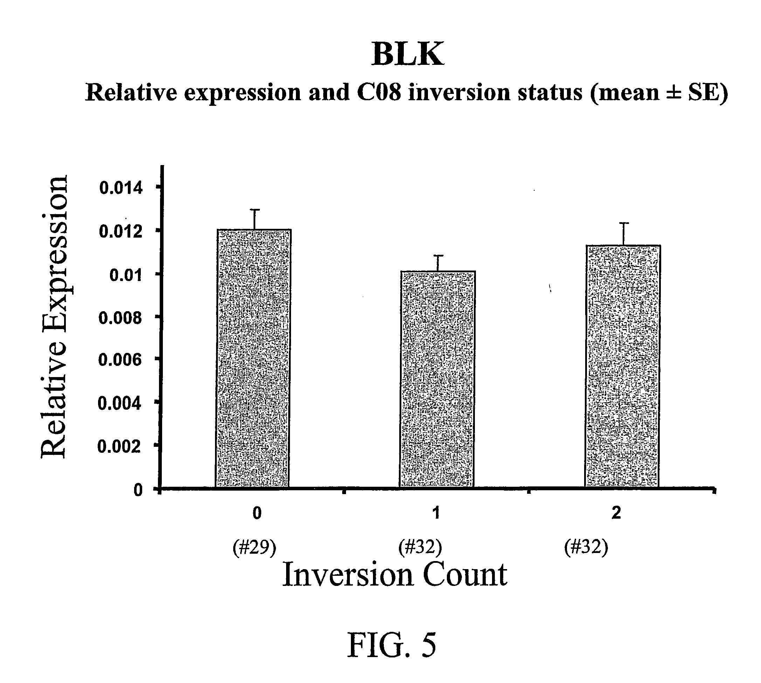

FIG. 5 shows a Bar-graph of the mean expression values for BLK (from FIGS. 2 and 3) for the 3 groups (with inversion counts of 0, 1, and 2; see Example 1). Each group is represented by a bar, and at the top of the bar the standard error is indicated. Also depicted are the results of association analyses where natural logarithm of expressions relative to β-actin on the inversion count. Regressing log-transformed relative expression on estimated number of inverted chromosomes gives : P-value = 0.46 and correlation coefficient $R^2$ = 0.006.

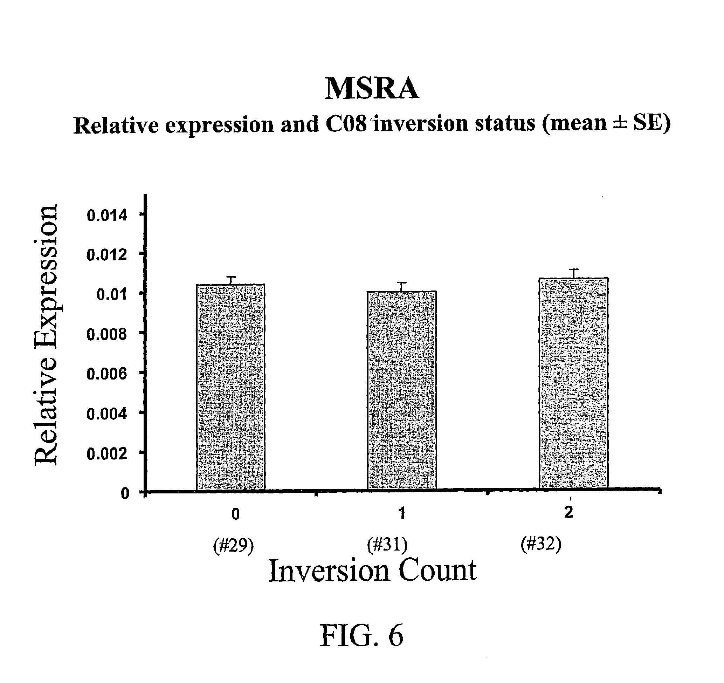

FIG. 6 shows a Bar-graph of the mean expression values for MSRA (from FIGS. 2 and 3) for the 3 groups (with inversion counts of 0, 1, and 2; see Example 1). Each group is represented by a bar, and at the top of the bar the standard error is indicated. Also depicted are the results of association analyses where natural logarithm of expressions relative to β-actin on the inversion count. Regressing log-transformed relative expression on estimated number of inverted chromosomes gives : P-value = 0.73 and correlation coefficient $R^2$ = 0.0012.

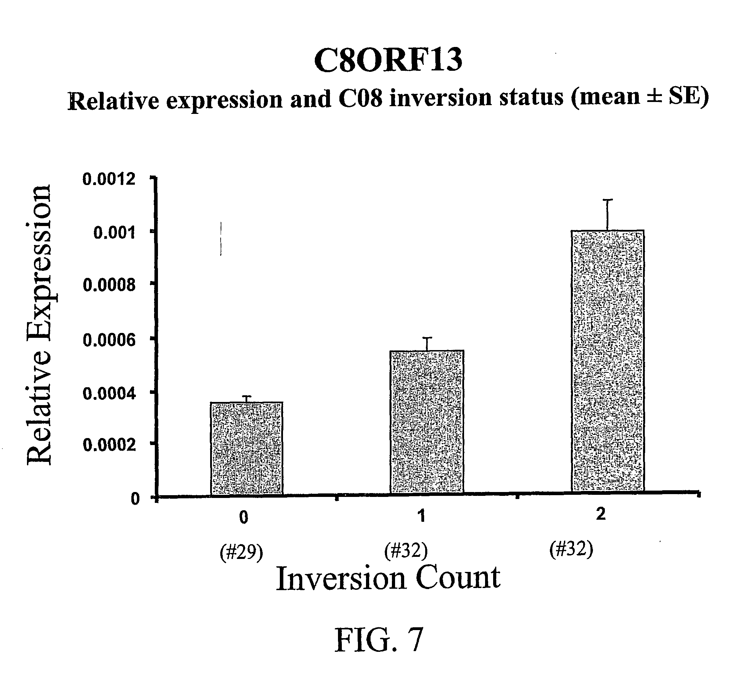

FIG. 7 shows a Bar-graph of the mean expression values for C80RF13 (from FIGS. 2 and 3) for the 3 groups (with inversion counts of 0, 1, and 2; see Example 1). Each group is represented by a bar, and at the top of the bar the standard error is indicated. Also depicted are the results of association analyses where natural logarithm of expressions relative to β-actin on the inversion count. Regressing log-transformed relative expression on estimated number of inverted chromosomes gives : P-value = 0.000000028 and correlation coefficient $R^2$ = 0.29.

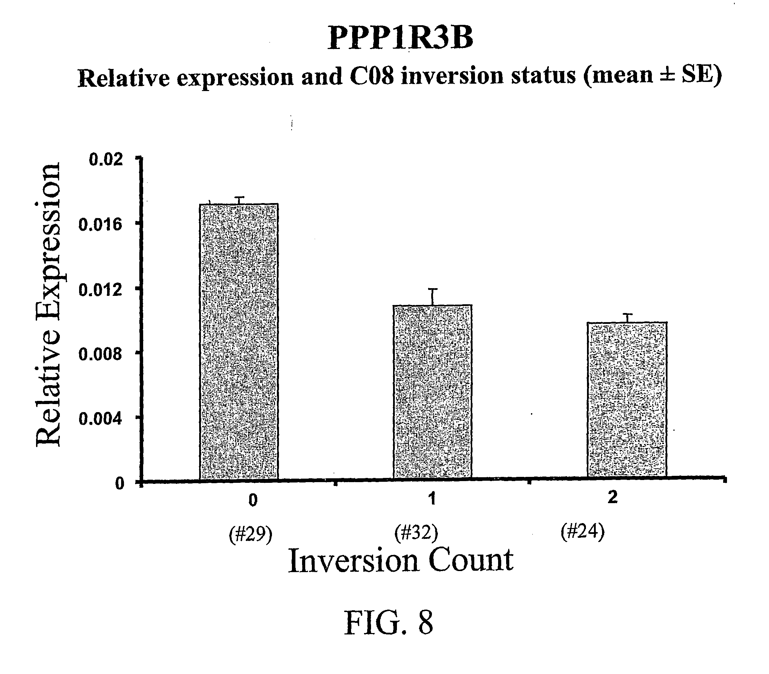

FIG. 8 shows a Bar-graph of the mean expression values for PPP1R3B (from FIGS. 2 and 3) for the 3 groups (with inversion counts of 0, 1, and 2; see Example 1). Each group is represented by a bar, and at the top of the bar the standard error is indicated. Also depicted are the results of association analyses where natural logarithm of expressions relative to β-actin on the inversion count. Regressing log-transformed relative expression on estimated number of inverted chromosomes gives : P-value = 0.0000011 and correlation coefficient $R^7$ = 0.25.

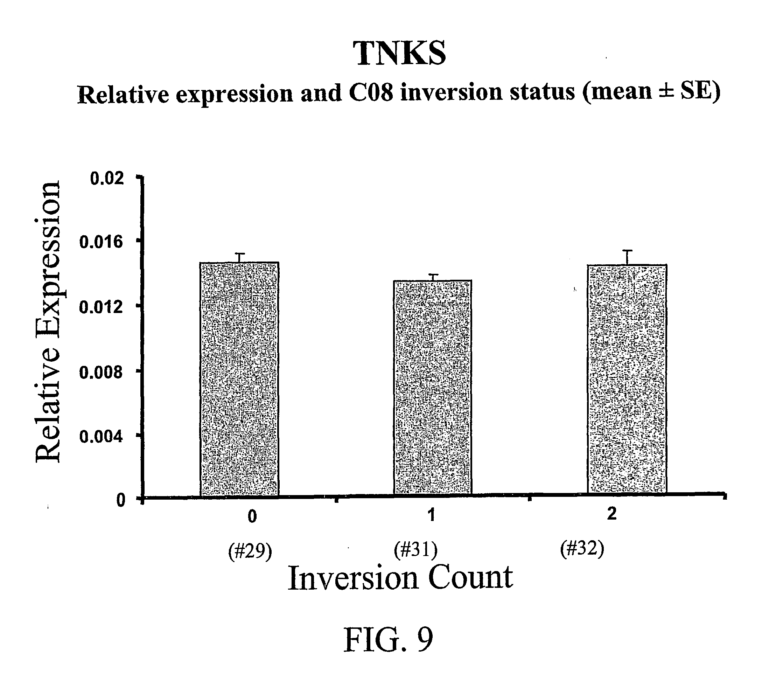

FIG. 9 shows a Bar-graph of the mean expression values for TNKS (from FIGS. 2 and 3) for the 3 groups (with inversion counts of 0, 1, and 2; see Example 1). Each group is represented by a bar, and at the top of the bar the standard error is indicated. Also depicted are the results of association analyses where natural logarithm of expressions relative to β-actin on the inversion count. Regressing log-transformed relative expression on estimated number of inverted chromosomes gives : P-value = 0.0635 and correlation coefficient $R^2$ = 0.4.

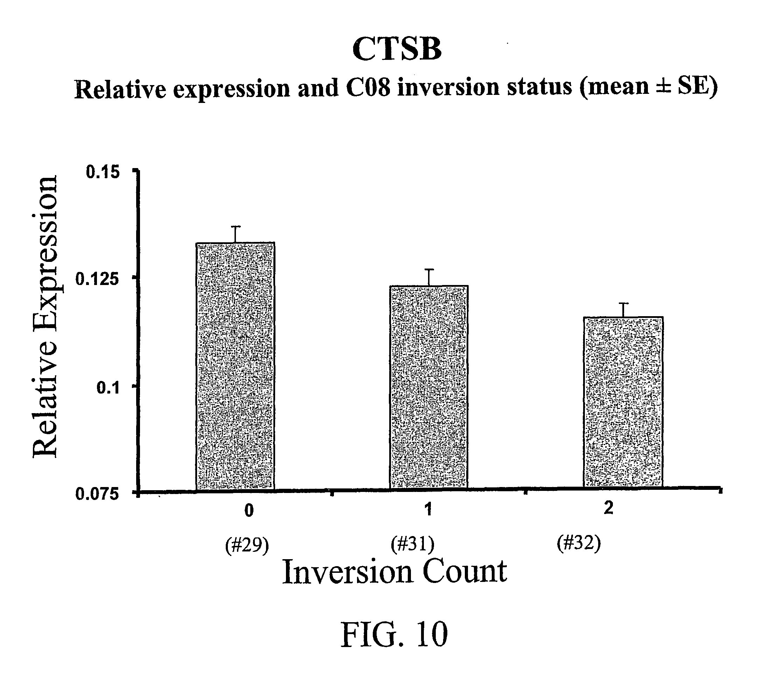

FIG. 10 shows a Bar-graph of the mean expression values for CTSB (from FIGS. 2 and 3) for the 3 groups (with inversion counts of 0, 1, and 2; see Example 1). Each group is represented by a bar, and at the top of the bar the standard error is indicated. Also depicted are the results of association analyses where natural logarithm of expressions relative to β-actin on the inversion count. Regressing log-transformed relative expression on estimated number of inverted chromosomes gives : P-value = 0.0014 and correlation coefficient $R^2$ = 0.108.

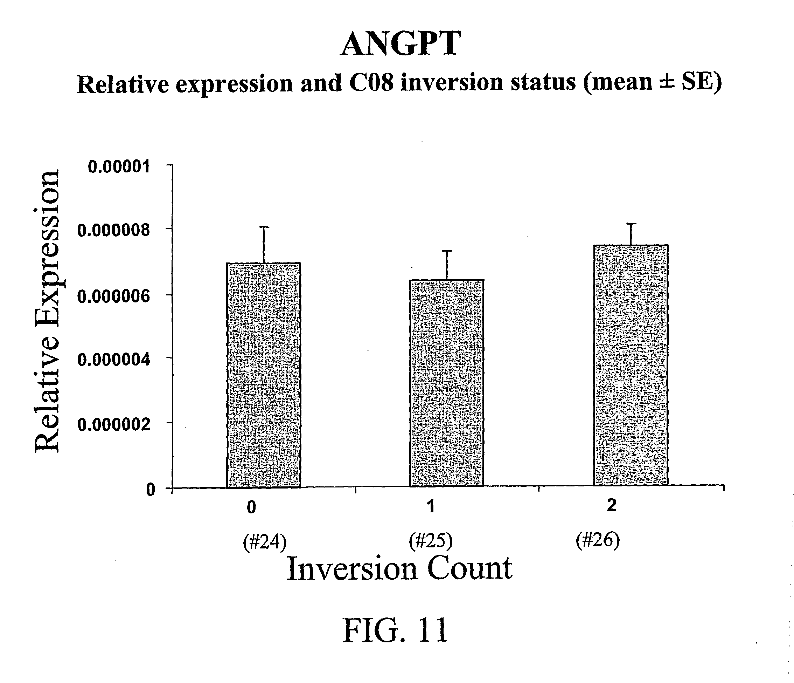

FIG. 11 shows a Bar-graph of the mean expression values for ANGPT (from FIGS. 2 and 3) for the 3 groups (with inversion counts of 0, 1, and 2; see Example 1). Each group is represented by a bar, and at the top of the bar the standard error is indicated. Also depicted are the results of association analyses where natural logarithm of expressions relative to β-actin on the inversion count. Regressing log-transformed relative expression on estimated number of inverted chromosomes gives P-value = 0.41 and correlation coefficient $R^2$ = 0.009.

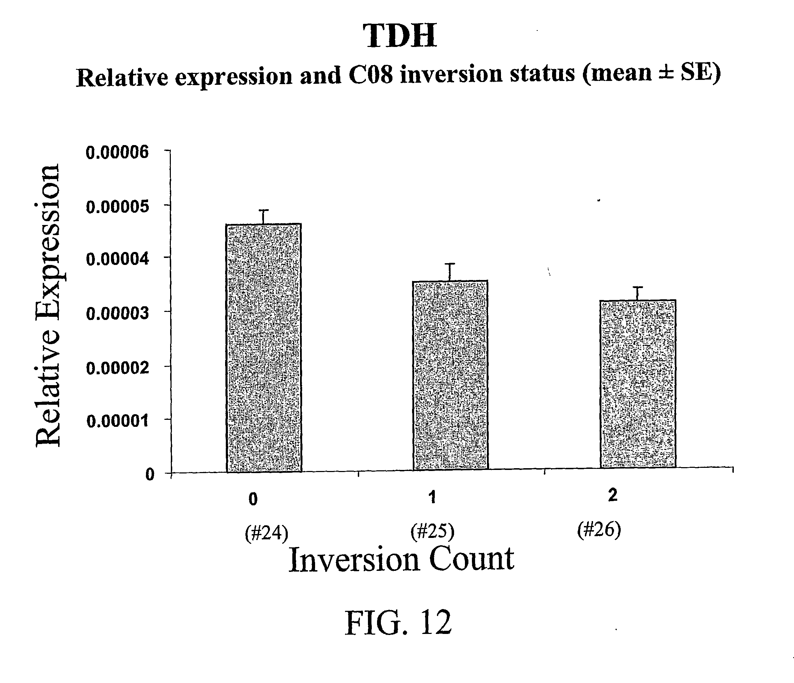

FIG. 12 shows a Bar-graph of the mean expression values for TDH (from FIGS. 2 and 3) for the 3 groups (with inversion counts of 0, 1, and 2; see Example 1). Each group is represented by a bar, and at the top of the bar the standard error is indicated. Also depicted are the results of association analyses where natural logarithm of expressions relative to β-actin on the inversion count. Regressing log-transformed relative expression on estimated number of inverted chromosomes gives P-value = 0.0003 and correlation coefficient $R^2$ = 0.16.

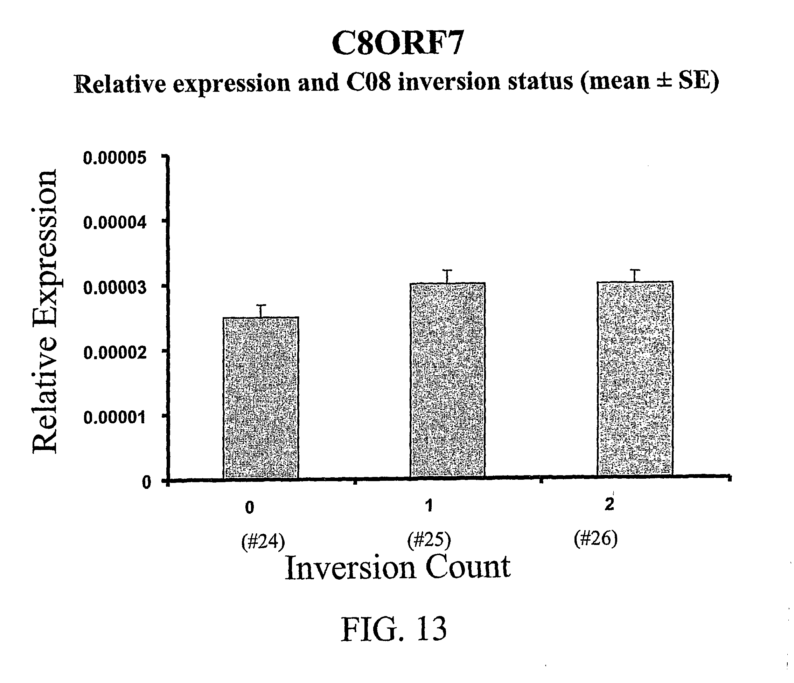

FIG. 13 shows a Bar-graph of the mean expression values for C80RF7 (from FIGS. 2 and 3) for the 3 groups (with inversion counts of 0, 1, and 2; see Example 1). Each group is represented by a bar, and at the top of the bar the standard error is indicated. Also depicted are the results of association analyses where natural logarithm of expressions relative to β-actin on the inversion count. Regressing log-transformed relative expression on estimated number of inverted chromosomes gives P-value = 0.025 and correlation coefficient $R^2$ = 0.06.

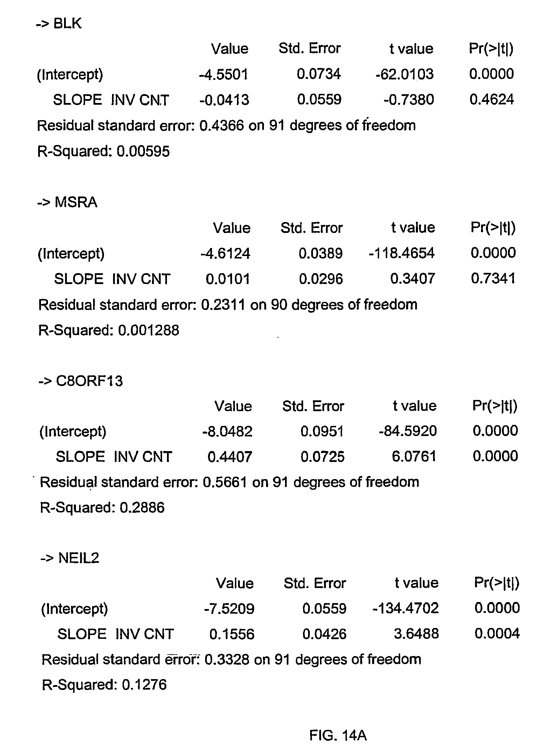

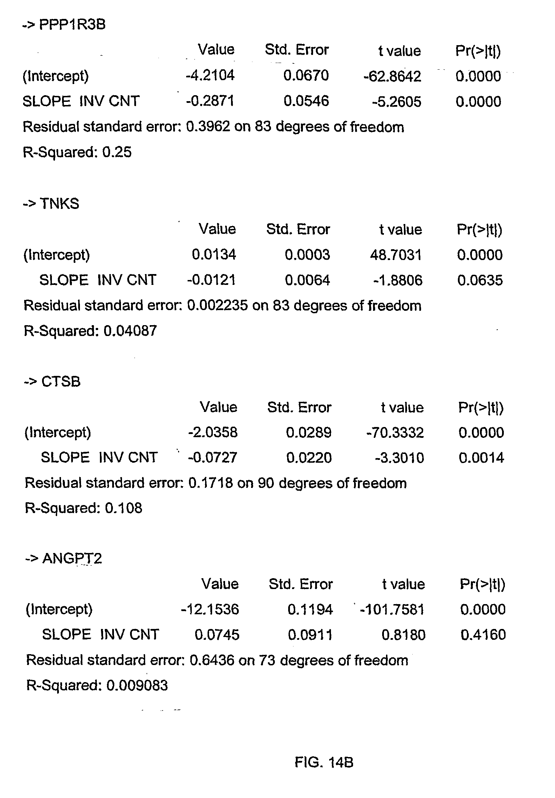

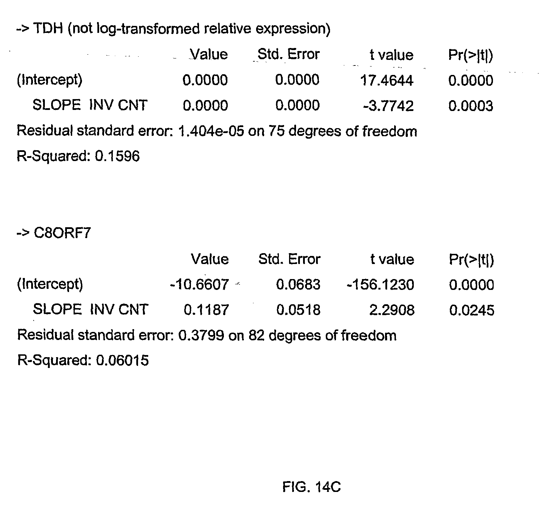

FIGS. 14A-14C show the output of the linear regression, showing the intercepts and slopes of expression value as a function of the inversion count. Also displayed are the standard error, and results of t-test (t-value and probability of observing the value obtained).

## DETAILED DESCRIPTION OF THE INVENTION

A description of preferred embodiments of the invention follows.

The present invention is based upon the discovery of the relationship between the orientation of the Inv8p23 polymorphism and gene expression. The orientation of the Inv8p23 inversion fragment is linked to psychiatric disorders, *e.g.*, anxiety disorder such as, for example, panic disorder, bipolar disorder and depression (see PCT application WO05040427A2, "Inversion on Chromosome 8p23 is Risk Factor For Anxiety Disorders, Depression and Bipolar Disorders.")

The orientation of the Inv8p23 polymorphism in an individual can be determined using surrogate markers. Examples of such markers are DG8S197, DG8S163, and DG8S132, although the person skilled in the art will recognize that other markers in linkage disequilibrium with the polymorphism can also be used to determine its orientation. The use of polymorphic markers to determine the status of the polymorphism is described in detail in WO05040427A2.

The invention provides important clues to the phenotypic effect and evolutionary history of the Inv8p23 inversion. Little is known about the effect of inversions in humans, but the role of inversions has been studied in drosophila, where inversion polymorphisms have been shown to be quite significant for adaptation from studies of frequency changes (seasonal and long-term frequency changes in response to environmental challenges), see Puig *et al.* (Puig, M. et al., 2004. Proc. Natl. Acad. Sci. USA , 101:9013-8) and Schaeffer *et al.* (Schaeffer, S. et al., 2003. Proc. Natl. Acad. Sci. USA, 100:8319-24), and references therein. Inversions, like single nucleotide mutations, are subjected to selection, and the molecular mechanisms underlying their maintenance in the human population is unknown, as it is for drosophila despite extensive studies. There are two main hypotheses aiming to explain their existence and maintenance at intermediate frequencies: A position effect of the inversion as the breakpoints are within or near genes that are affected 3, or an effect of co-adaptation, *e.g.*, through the reduction of recombination between the different orientations different but favorable combinations of gene variant distributions develop over time on the two orientations (Puig, M. et al., 2004. Proc. Natl. Acad. Sci. USA , 101:9013-8; Charlesworth, B., 1974. Genet Res., 23:259-80).

There is increasing evidence for a role of genomic rearrangements in complex disorders. Described herein are gene expression data related to a common inversion polymorphism on chromosome 8p23 originally reported by Giglio et al. (Am. J. Hum. Genet., 68:874-83). FISH measurements indicated that the less frequent form of the inversion was in association to Panic Disorder (PD) in Iceland (see PCT application WO05040427A2, "Inversion on Chromosome 8p23 is Risk Factor For Anxiety Disorders, Depression and Bipolar Disorders."). Joint analysis of FISH and genotype data identified excellent surrogate markers for the inversion status. The present invention relates to the finding that, after quantitation of RNA from blood and lymphoblast cell lines, the expression of several genes within the inversion region is strongly associated with the orientation of the segment. Thus, the results indicate a role of the inversion polymorphism on 8p23 in the etiology of complex phenotypes.

The present invention relates to methods for determining the orientation of the Inv8p23 inversion fragment, treatment outcome of psychiatric diseases, *e.g.*, anxiety disorder such as, for example, panic disorder and bipolar disorder, and drug responsiveness for psychiatric disorders based on gene expression profiles of genes in the Inv8p23 genomic region. The methods rely on the identification of genes that are differentially expressed in samples obtained from patients. The particular genes, herein referred to as "informative genes," are identified in samples obtained from patients suspected of having a psychiatric disorder. Informative genes can be identified, for example, by determining the ratio of gene expression in a sample from an individual with a known genotype for the Inv8p23 site and a sample from an individual with the opposite genotype at the Inv8p23 site. A ratio of 1.0 would indicate the gene is expressed at the same level in both samples. Ratios greater than one indicate increased expression of one orientation, whereas ratios less than one indicate reduced expression relative to the opposite orientation.

A subset or all informative genes can be assayed for gene expression in order to generate an "expression profile". As used herein, an "expression profile" refers to the level or amount of gene expression of one or more informative genes in a given sample of cells at one or more time points. A "reference" expression profile is a profile of a particular set of informative genes under particular conditions such that the expression profile is characteristic of a particular condition. For example, a reference expression profile that quantitatively describes the expression of the informative genes listed in FIGS. 4-13 can be used as a reference expression profile. In one embodiment, expression profiles are comprised of the ten informative genes that exhibit differential expression, and provide sufficient power to predict the orientation of the Inv8p23 inversion fragment. Other embodiments can include, for example, expression profiles containing about 5 informative genes, about 25 informative genes, about 100 informative genes, or any number of genes in the range of about 5 to about 400 informative genes. The informative genes that can be used in the expression profile can be genes that are indicative of either orientation. The particular set of informative genes used to create an expression profile can be, for example, the genes that exhibit the greatest degree of differential expression, or they can be any set of genes that exhibit some degree of differential expression and provide sufficient power to accurately predict the orientation of the Inv8p23 inversion fragment. The genes selected are typically those that have been determined to be differentially expressed, wherein the differential expression is relative to a particular orientation of Inv8p23. By comparing samples from patients with these reference expression profiles, the patient's genotype at the Inv8p23 locus can be determined. The orientation is indicative of a psychiatric disorder, *e.g.*, depression or anxiety disorder, such as, for example, panic disorder and bipolar disorder. For example, the inverted orientation has been shown to be associated with psychiatric disorders in the Icelandic population.

The generation of an expression profile requires both a method for quantitating the expression from informative genes and a determination of the informative genes to be screened. The present invention describes screening specific changes in individuals that affect the expression levels of gene products in samples. As used herein, "gene products" are transcription or translation products that are derived from a specific gene locus. The "gene locus" includes coding sequences as well as regulatory, flanking and intron sequences. Expression profiles are descriptive of the level of gene products that result from informative genes present in cells. Methods are currently available to one of skill in the art to quickly determine the expression level of several gene products from a sample of cells. For example, short oligonucleotides complementary to mRNA products of several thousand genes can be chemically attached to a solid support, *e.g.*, a "gene chip," to create a "microarray." Specific examples of gene chips include Hu95GeneFL (Affymetrix, Santa Clara, CA) and the 6800 human DNA gene chip (Affymetrix, Santa Clara, CA). Such microarrays can be used to determine the relative amount of mRNA molecules that can hybridize to the microarrays (Affymetrix, Santa Clara, CA). This hybridization assay allows for a rapid determination of gene expression in a cell sample. Alternatively, methods are known to one of skill in the art for a variety of immunoassays to detect protein gene expression products. Such methods can rely, for example, on conjugated antibodies specific for gene products of particular informative genes.

Although the reference expression profile is typically representative of a specific orientation of the Inv8p23 genomic region, the person skilled in the art will realize that the present invention is also compatible with a reference expression profile that is not representative of either specific orientation of the Inv8p23 genomic region, but rather represents a combination, i.e. one copy of each orientation. The important thing is to compare the expression profile of an individual to an expression profile that is representative of a known orientation, or a known mixture thereof. However, the person skilled in the art will also realize that the expression profile is preferentially representative of one orientation or the other.

Informative genes can also be identified *ex vivo* in cells derived from patient samples. For example, a tissue sample can be obtained from an individual and cells derived from this sample can be cultured *in vitro*. Expression profiles of informative genes can be obtained from sample-derived cells.

Once informative genes have been identified, polymorphic variants of informative genes can be determined and used in methods for detecting disorders in patient samples based on which polymorphic variant is present in the sample (*e.g.*, through hybridization assays or immune detection assays using antibodies specific for gene products of particular polymorphic variants). Genetic markers are particular "alleles" at "polymorphic sites". A nucleotide position at which more than one sequence is possible in a population (either a natural population or a synthetic population, *e.g.*, a library of synthetic molecules) is referred to herein as a "polymorphic site". Where a polymorphic site is a single nucleotide in length, the site is referred to as a single nucleotide polymorphism ("SNP"). For example, if at a particular chromosomal location, one member of a population has an adenine and another member of the population has a thymine at the same position, then this position is a polymorphic site, and, more specifically, the polymorphic site is a SNP. Polymorphic sites can allow for differences in sequences based on substitutions, insertions or deletions. Each version of the sequence with respect to the polymorphic site is referred to herein as an "allele" of the polymorphic site. Thus, in the previous example, the SNP allows for both an adenine allele and a thymine allele. "Markers" are genetic elements, *e.g.*, SNPs, genes, polymorphisms, drug resistance, restriction sites, etc., or combinations of genetic elements, *e.g.*, haplotypes, that can be used to indicate a particular characteristic.. For example, if a particular SNP is demonstrated to be associated with a particular phenotype, then the detection of the particular SNP is indicative of the particular phenotype. In this example, the SNP is used as a marker. For the purposes of the present invention, the Inv8p23 inversion fragment represents a polymorphic site that is a marker for psychiatric disorders.

Alternatively, the approach described above can be used to verify the utility of informative genes identified in cultured cells. Once identified, informative genes could be verified as to their predictive ability in more genetically diverse populations, thus ensuring the utility of the predictive power of these informative genes in populations in addition to the genetically isolated population of, *e.g.*, Iceland.

The "genetic isolation" of the Icelandic population implies a low degree of allelic variation among individuals. This circumstance reduces the background in screening for differences in a population. In "genetically diverse" populations, many differences appear between individuals that might contribute to the same trait. For example, an examination of individuals responsive for asthma drug treatment might produce a finite yet large number of genetic differences with respect to non-responsive individuals. However, in a genetically diverse population, a great majority of these genetic differences are "artifactual" or background "noise signals" detected because of the diversity of the population. For a genetically isolated population, fewer differences would be expected to be found between the two groups, providing a higher probability that the differences that are discovered are likely to be directly related to the trait in question, in this case, responsiveness to asthma drug treatment. Once determined in a genetically isolated environment, the utility of informative genes and expression profiles based on those informative genes can be verified for more general use in a genetically diverse population.

An individual at risk for or to be diagnosed with a psychiatric disorder, *e.g.*, an anxiety disorder, PD or a comorbid disorder is an individual who has at least one inverted allele (Inv8p23) of the inversion polymorphism on chromosome 8. This allele can be identified by the methods described herein and in the PCT application WO05040427A2. The methods described herein can therefore serve as predictors of susceptibility to or as an indicator of a psychiatric disorder, anxiety disorder, PD or a comorbid disorder. As used herein, a "comorbid disorder" refers to a disorder existing simultaneously with and usually independently of another medical condition, *e.g.*, PD. Examples of disorders comorbid with PD include, but are not limited to, depression, bipolar disorder (BPD; also known as manic-depressive illness), obsessive-compulsive disorder (OCD), histrionic personality disorder, family denial and dysfunction, hypercholesterolemia and substance abuse.

In addition to the use of the differential expression data provided herein for use in methods for diagnosing psychiatric disorders, the differentially expressed genes are likely candidates for drug targets. One of skill in the art would appreciate the fact that since the differential expression correlates with a marker known to associate with psychiatric disorders, it is likely that altered expression of one or more of these genes plays a direct role in causing the disease. Therefore, methods for treating the disease will be available to one of skill in the art by modulating the expression of an informative gene in an individual suffering from a psychiatric disorder.

One of skill in the art will recognize that reagents necessary to utilize the methods described herein can be contained in a kit. Such reagents as described are either commercially available (*e.g.*, buffered solutions, chemical reagents) or produced by methods known in the art (*e.g.*, oligonucleotides, antibodies, ligands for detection). Thus, one of skill in the art would recognize that a kit can be produced containing in appropriate compartments, for example, all reagents, probes, and materials necessary for to allow for the practice of the methods described herein.

The present invention relates also to methods for predicting efficacy of drug treatment of psychiatric disorders, anxiety disorders, PD and comorbid disorders. Current methods of treating such disorders with drugs have significant risks of substantial side effects. Thus, determining whether an individual will be effectively treated with a particular drug treatment will be useful. Drugs useful in treating psychiatric disorders include, for example, Amine Reuptake Inhibitors, *e.g.*, Selective Serotonin Reuptake Inhibitors (*e.g.*, fluoxetine, sertraline, paroxetine, fluvoxamine), Selective Norepinephrine Reuptake Inhibitors (*e.g.*, desipramine, maprotiline), Combined Serotonin-Norepinephrine Reuptake Inhibitors (*e.g.*, Selective (*e.g.*, venlafaxine) or Non-selective (*e.g.*, tertiary amine tricyclics, nortriptyline), Combined Dopamine-Norepinephrine Reuptake Inhibitors (*e.g.*, Selective (*e.g.*, bupropion)); Inhibitors of Enzymatic Metabolism, *e.g.*, Irreversible/nonselective Monoamine Oxidase Inhibitors (*e.g.*, phenelzine, tranylcypromine, isocarbozazid), Reversible/selective Inhibitor of Monoamine Oxidase-A (*e.g.*, moclobimide); Receptor Antagonists, *e.g.*, 5-HT 2A receptor antagonist (*e.g.*, Nonselective (*e.g.*, trazodone)), Combined 5-HT 2A antagonist with Serotonin Reuptake Inhibition (*e.g.*, Nonselective (*e.g.*, nefazodone), tricyclics, and Combined 5-HT 2A, 5-HT 2C and alpha-2 antagonists (*e.g.*, Nonselective (*e.g.*, mirtazipine)). Although psychiatric disorders, *e.g.*, depression, have been treated by these drugs for several years, a significant fraction of patients are non-responsive or show little effect of the treatment. As there are risks associated with methods for treating psychiatric disorders, identification of patients that will be responsive to treatment is important. Methods described herein can be used to identify markers that are associated with drug responsiveness.

The determination of drug responsiveness is accomplished by obtaining the gene expression profile for one or more informative genes that are useful in determining the orientation of the Inv8p23 inversion fragment. The present invention is directed, for example, in determining drug responsiveness of a human patient for a drug used to treat psychiatric disorder. A "responder" population is identified and associated with a particular orientation, *e.g.*, the inverted orientation, of the Inv8p23 inversion fragment. Therefore, using a gene expression profile to determine the orientation of the inversion fragment identifies whether a person is a responder to a particular drug. The opposite orientation would be indicative of "non-responders".

The invention will be further described with reference to the following nonlimiting examples.

EXAMPLES

Example 1.

Preliminary analysis of existing Affymetrix chip gene expression data revealed potential differences with regard to gene expression of genes associated with the orientation of the Inv8p23 genomic region (*e.g.*, GATA-4 gene). Rather than relying on chip data to further check this idea, TaqMan® assays were performed to quantitate RNA isolated from blood. By testing the relationship between the orientation of the inversion and expression of 10 genes at chromosome 8p23, highly significant association of expression to the orientation of the inverted segment for a majority of the genes was found. Therefore, the inversion orientation is associated with the expression of individual genes within or near the inverted segment, with the consequence that the different orientations have their own particular gene expression patterns.

Selection of Study Samples

A list of RNA samples that had all been isolated using the same method (see below) from the blood of individuals who had been genotyped with markers from the inversion region was obtained. From this list, individuals with high quality RNA available (isolated from blood for chip expression studies in other projects at deCODE genetics, Inc.) who had been genotyped with surrogate markers (DG8S197, DG8S163, DG8S132) were selected. The inversion count (0, 1, or 2 inverted chromosomes) was then estimated from the genotypes for the above markers. Individuals homozygous for "haplotype 1" (DG8S197 0 DG8S163 0 DG8S132 0) received an inversion count of 2, those homozygous for "haplotype 0" (DG8S197 3 DG8S163 7 DG8S132 0) received an inversion count of 0, and those heterozygous for all markers received and inversion count of 1. Below the prevalence of the alleles for these markers on both forms are displayed:

|          |   | Inverted Form | Common Form |
|----------|---|---------------|-------------|
| DG8S197  | 1 | 85 %          | 11 %        |
| DG8S163  | 0 | 94 %          | 17 %        |
| DG8S132  | 0 | 94 %          | 6%          |

After determining the inversion count as described above, approximately 30 of each inversion count category (0, 1, 2) were studied by expression profiling. 14 assays have been run, and, of these, 10 gave strong enough expression to test the idea.

Gene Expression Studies.

Preparation of RNA

RNA was isolated from blood using Qiagen Rneasy MIDI kit according to the manufacturer's instructions. Concentration and quality of the RNA was determined with Agilent 2100 Bioanalyzer (Agilent Technologies). See Example 2 for standard operating procedures for RNA preparation and quality control.

Preparation of cDNA

Total RNA was converted to cDNA using the TaqMan Reverse Transcription System (Applied Biosystems), primed with random hexamers.

Taqman expression assay

Commercially available TaqMan® gene expression assays (Applied Biosystems) were used for the genes under investigation. The PCR was carried out with TaqMan® Universal Master Mix (Applied Biosystems) using 15 ng cDNA, 9 nmol each primer and 2.5 nmol each probe in a 10 $\mu$L total reaction volume. Each sample was run in duplicate for 10 min at 95°C followed by 40 cycles of 15s at 95$\beta$C and 1 min at 60$\beta$C on an ABI Prism 7900HT Sequence Detection System. Quantification was carried out according to the manufacturer's recommendations (User Bulletin #2, ABI Prism 7700 Sequence Detection System December 11, 1997 (updated 10/2001)).

Data Analysis

First, the means and variances for the two groups were calculated, (inversion count = 0 and inversion count = 2), and statistical tests were performed to evaluate the significance of observed differences between the mean expression values for these two groups. The data and results are displayed in FIGS. 2 and 3. Note that when no value is given for an expression measurement the measurement failed and was not used in calculations. Linear regression analysis was also performed on the whole dataset for each gene to utilize information from those with inversion count = 1 in addition to those with inversion counts of 0 and 2. The results of these analyses are depicted graphically in FIGS. 4-13, and the output of the linear regression program is shown in FIGS. 14A-14C. Prior to regression analysis the data were checked for consistency and in case of disagreements between individual measurements in the duplicate (when the larger value was larger than 1.5 x the lower value) both measurements were discarded prior to analysis. For these studies, a natural logarithm transformation of the data (expression values relative to β-actin) was used to render the distribution closer to a normal distribution, except in two cases where the data had a nearly normal distribution such that transformation was not required. Then, the expression values on the inversion count were regressed using S-plus version 5.

Results

For most of the genes there is a strong correlation between expression and the inversion count. Visual inspection of FIGS. 4-14 shows this clearly, and this is confirmed by both analyzing the difference between the homozygous groups (FIGS. 2 and 3) and the regression analysis of all three groups (FIGS. 4-13). The most significant associations are for CTSB, TDH, PPP1R3B, C8ORF13, C8ORF7, and NEIL2 with the expression of CTSB, TDH, PPP1R3B lower on the inverted form than the common form and the expression of C8ORF13, C8ORF7, and NEIL2 higher on the inverted form than the common form. BLK and TNKS also show distinct patterns where the expression for heterozygotes is lower than for the homozygous groups of both orientations; thus they do not give significant results in the linear regression.

The fact that 10 genes from the 8p region show very strong correlation between expression and the inversion orientation is significant for other genes in this region. It is very likely that a large fraction of the genes that remain to be analyzed will also do so. The results described herein make it clear that the inversion orientation is associated to particular patterns of expression.

Example 2.

Isolation of RNA from whole blood using RNeasy MIDI kit

Procedure

1. Scanning and tracking of samples

Before samples were processed, they were scanned to Sample Manager as sample type 12 (clean RNA). Serum samples were scanned as type 04 (serum) in Merck Serum box. Phlebotomists filled in a tracking form for each sample with date and time of blood collection. When samples arrived in the lab, these tracking forms were filled in with information regarding receipt time, receiving tech, processing tech and lot number of all reagents and time when EL buffer was added and cells were lysed in RLT buffer.

2. Preparation

Centrifuges were cooled to 4°C before starting. β-Mercaptoethanol (BME) was added to Buffer RLT before use (10 μL BME per 1 mL of Buffer RLT). The solution is stable for up to one month.

Buffer RPE was supplied as a concentrate. Four volumes of ethanol (96-100%) were added as indicated on the bottle to obtain a working stock solution.

DNase I stock solution was prepared before using the RNase-free DNase set for the first time. The solid DNase I was dissolved in 550 μL of the RNase-free water provided. The solution was mixed gently by inverting the tube. For long term storage of DNase I, the solution was divided into single-use aliquots and stored at -20°C for up to 9 months. Thawed aliquots can be stored at 2-8°C for up to 6 weeks.

3. Standard Procedure Followed for Lysis and RNA Isolation

1. Mix 9.0 mL whole blood with 40.0 mL of Buffer EL in a 50 mL screw cap tube. It is preferable to use 5 volumes of Buffer EL per 1 volume of blood, but 40.0 mL of Buffer EL can be used to lyse 9.0 mL of whole blood in a single 50 mL tube, with small increase in the incubation time.
2. Incubate for 10-15 min on ice. Mix by vortexing briefly twice during the incubation. The cloudy suspension becomes translucent during incubation, indicating lysis of red blood cells. When multiple samples are processed, make sure the incubation time does not exceed 20 minutes, otherwise the leukocytes begin to lyse and RNA yield will decrease.
3. Centrifuge at 400 x g for 10 min at 4°C. Discard the supernatant. Save the leukocyte pellet.
4. Add 20.0 mL Buffer EL to the leukocyte pellet. Resuspend cells by vortexing briefly.

5. Centrifuge at 400 x g for 10 min at 4°C. Discard the supernatant. Save the leukocyte pellet. After centrifuging, heat the centrifuge to 20-25°C.

6. Add 4.0 mL of Buffer RLT. Vortex to mix and remove clumps. Ensure that β-Mercapto-ETOH has been added to Buffer RLT before use.

7. Homogenize the leukocytes using a rotor-stator homogenizer for at least 45s at maximum speed until the sample is uniformly homogeneous. Wash homogenizer in DEPC-water for 10 s and then in RLT for 10 s after each sample.

8. Add 4.0 mL of 70% ethanol to the lysate, and mix thoroughly by shaking vigorously/vortexing. Do not centrifuge. Apply half of the sample to an RNeasy midi column placed in a 15 mL centrifuge tube. Close the tube gently, and centrifuge for 5 min at 3220 x g (max). Discard the flow-through.

9. Apply remaining half of the sample to the same midi column, and centrifuge as above. Discard the flow-through. Reuse the centrifuge tube in step 10.

10. Pipet 2.0 mL of Buffer RW1 into the RNeasy column. Centrifuge for 5 min at 3220 x g to wash. Discard the flow-through. Reuse the centrifuge tube in step 12.

11. During centrifugation, prepare RNase-Free DNase I solution. Calculate the amount needed as:

$$(20 \text{ μL DNase} \times n+1) + (140 \text{ μL Buffer RDD} \times n+1) \qquad n = \text{ sample number.}$$

Mix by gently by flicking or inverting the tube. Do not vortex.

12. Make sure that the membrane is dry. If it is not, spin for 5 more minutes. Pipet 160 μL DNase I mix directly onto the RNeasy column, and place on the bench top (20-30°C) for 15 min. Make sure to pipet the DNase I mix directly onto the RNeasy silica-gel membrane. DNase digestion will be incomplete if part of the mix sticks to the walls or the O-ring of the column.

13. Pipet 2.0 mL Buffer RW1 into the RNeasy column, and place on the bench top for 5 min. Centrifuge for 5 min at 3220 x g. Discard the flow-through.

14. Add 2.5 mL Buffer RPE to the RNeasy column. Ensure that ETOH has been added to Buffer RPE before using a new bottle for the first time. Centrifuge for 2 min at 3220 x g to wash the column. Reuse the centrifuge tube in step 15.

15. Add another 2.5 mL Buffer RPE to the RNeasy column. Centrifuge for 5 min at 3220 x g to dry the RNeasy silica-gel membrane. It is important to dry the RNeasy membrane. This centrifugation ensures that no ethanol is carried over to affect elution. Carefully remove the RNeasy column from the centrifuge tube so the column does not contact the flow-through.

16. To elute, transfer column to a new 15 mL collection tube. Pipet 150 μL of RNase-free water onto the RNeasy silica-gel membrane. Let stand for 1 min, then centrifuge for 3 min at 3220 x g.

17. Repeat the elution with extra 100 μL of RNase-free water (total 250 μL). Put samples on ice. Move samples to 1.8 mL RNase free tube.

18. Put samples straight into -80°C.

4. Procedure Followed for Packard handling and spectrophotometer measurement

1. Start by doing a daily washing program for the Packard. Go to desktop and press "daily wash". Press "executing test" and the program will start.

2. Place 12 Eppendorf tubes, 11 test samples and one control (Human Universal Reference Total RNA, 50ng/μL) on a tray and open them. Place the tray in the right position on the robot.

3. An empty UV-plate is placed in the right position on the robot.

4. Check the robot for tips, DEPC- water and ensure that there is enough of TE-buffer in an appropriate box.

5. Go back to desktop and open program "Spectrophotometer dilution". When everything is ready press "Execute Test".

6. The robot now draws 4 μL from each sample and transfers them to the UV-plate.

7. The robot then draws 196 μL of the TE buffer and adds it to each sample in the UV-plate. Finally, the robot puts 3 blanks on the plate.

8. When the robot is done the UV-plate is ready.

9. Put samples directly on ice in the same order as they were transferred to the plate. Try to work as quickly as possible.

10. Measure plate on a spectrophotometer at 280 and 260 nm.

5. Procedure used to determine the Concentration of RNA that was below 0.2 μg/μL

1. Add 1/10th volume 3.0 M NaAc, 2 volumes of 100% ethanol, mix and precipitate at -20°C for 1 hour.
2. Microcentrifuge at maximum speed and 4°C for 20 minutes, decant supernatant.
3. Wash once with 1 mL 75% ethanol.
4. Centrifuge for 5 min at 4°C. Air dry.
5. Re-suspend in nuclease-free water so the end concentration will be around 0.2 μg/μL.
6. Re-measure the samples on the spectrophotometer (see section 4).

6. Procedure used for Sample Preparation for Bioanalyzer

1. Dilute sample in RNase free water and mix well. The dilution depends on the expected RNA yield (see Reagent Kit Guide, RNA 6000 Nano Assay). Place the sample on ice.
2. Use Human Universal Reference Total RNA as control (50ng/μL) on each chip.
3. Heat RNA 6000 ladder, samples and control at 70°C for 2 minutes and put on ice for at least 5 minutes.
4. Spin at 12000 x G for 20 seconds and keep on ice until measured.

**Claims**

1. A method for diagnosing a susceptibility to a psychiatric disorder in an individual, the method comprising:

    a) obtaining a gene expression profile of one or more informative genes from a sample obtained from an individual to be diagnosed; and
    b) comparing the gene expression profile of the sample with a reference gene expression profile, wherein the reference profile is representative of a specific orientation of the Inv8p23 genomic region,

    wherein comparison between the sample expression profile and the reference expression profile is indicative of the susceptibility a psychiatric disorder.

2. The method of Claim 1, wherein the psychiatric disorder is an anxiety disorder.

3. The method of Claim 2, wherein the anxiety disorder is panic disorder or bipolar disorder.

4. The method of any of the preceding claims, wherein the reference profile is representative of the inverted orientation of the Inv8p23 genomic region.

5. The method of any of the preceding claims, wherein the psychiatric disorder is comorbid with a disorder selected from the group consisting of: depression, bipolar disorder, obsessive-compulsive disorder, histrionic personality disorder, family denial and dysfunction, hypercholesterolemia and substance abuse.

6. The method of Claim 5, wherein the comorbid disorder is depression, bipolar disorder or hypercholesterolemia.

7. The method of any of the preceding claims, wherein the gene expression profile comprises the gene expression profile of one or more genes selected from the group consisting of those genes listed in FIGS. 1A-1C.

8. The method of Claim 7, wherein the gene expression profile comprises the gene expression profile of one or more genes selected from the group consisting of: NEIL2, BLK, MSRA, C80RF13, PPP1R3B, TNKS, CTSB, ANGPT, TDH and C80RF7.

9. The method of any of preceding claims, wherein the gene expression profile is obtained using a hybridization assay to oligonucleotides contained in a microarray.

10. The method of any of preceding claims, wherein the gene expression profile is obtained by detecting the protein products of the genes.

**11.** The method of Claim 10, wherein antibodies capable of specifically binding protein products of the genes are used to detect the protein products of the genes.

**12.** A method of predicting the efficacy of drug treatment of a psychiatric disorder in an individual, the method comprising:

a) obtaining a gene expression profile of one or more of informative genes from a sample obtained from the individual to be treated; and
b) comparing the gene expression profile of the sample with a reference gene expression profile, wherein the reference profile is representative of a specific orientation of the Inv8p23 genomic region,

wherein comparison between the sample expression profile and the reference expression profile is indicative of the efficacy of the drug treatment.

**13.** The method of Claim 12, wherein the psychiatric disorder is an anxiety disorder.

**14.** The method of Claim 13, wherein the anxiety disorder is panic disorder or bipolar disorder.

**15.** The method of any of Claims 12-14, where the psychiatric disorder is comorbid with a disorder selected from the group consisting of: depression, bipolar disorder, obsessive-compulsive disorder, histrionic personality disorder, family denial and dysfunction, hypercholesterolemia and substance abuse.

**16.** A method of predicting the orientation of Inv8p23 genomic region in an individual, the method comprising:

a) obtaining a gene expression profile of one or more informative genes from a sample obtained from an individual to be diagnosed; and
b) comparing the gene expression profile of the sample with a reference gene expression profile, wherein the reference profile is representative of a specific orientation of the Inv8p23 genomic region,

wherein comparison between the sample expression profile and the reference expression profile is indicative of said orientation.

**17.** The method of claim 16, wherein the reference profile is representative of the inverted orientation of the Inv8p23 genomic region.

**18.** The method of claim 16-17, wherein the gene expression profile comprises the gene expression profile of one or more genes selected from the group consisting of those genes listed in FIGS. 1A-1C.

**19.** The method of Claim 16-18, wherein the gene expression profile comprises the gene expression profile of one or more genes selected from the group consisting of: NEIL2, BLK, MSRA, C80RF13, PPP1R3B, TNKS, CTSB, ANGPT, TDH and C80RF7

**20.** The method of any of claims 16-19, wherein an inverted orientation of the Inv8p23 genomic region is indicative of the susceptibility of a psychiatric disorder or a comorbid disorder associated with the psychiatric disorder.

**Patentansprüche**

**1.** Verfahren zur Diagnose einer Anfälligkeit für eine psychiatrische Störung einer Person, wobei das Verfahren umfasst:

a) Erhalten eines Genexpressionsprofils eines oder mehrerer informativer Gene von einer Probe, die von einer zu diagnostizierenden Person erhalten wird; und
b) Vergleichen des Genexpressionsprofils der Probe mit einem Referenzgenexpressionsprofil, wobei das Referenzprofil für eine spezifische Orientierung der genomischen Inv8p23-Region repräsentativ ist,

wobei ein Vergleich zwischen dem Probenexpressionsprofil und dem Referenzexpressionsprofil die Anfälligkeit für eine psychiatrische Störung anzeigt.

**2.** Verfahren nach Anspruch 1, wobei die psychiatrische Störung eine Angststörung ist.

**3.** Verfahren nach Anspruch 2, wobei die Angststörung eine Panikstörung oder bipolare Störung ist.

**4.** Verfahren nach einem der vorangehenden Ansprüche, wobei das Referenzprofil für die invertierte Orientierung der genomischen Inv8p23-Region repräsentativ ist.

**5.** Verfahren nach einem der vorangehenden Ansprüche, wobei die psychiatrische Störung mit einer Störung komorbid ist, die ausgewählt ist aus der Gruppe bestehend aus: Depression, bipolarer Störung, Zwangsstörung, histrionischer Persönlichkeitsstörung, Familienentzug und Störung des familiären Gefüges, Hypercholesterolämie und Substanzenmissbrauch.

**6.** Verfahren nach Anspruch 5, wobei die komorbide Störung Depression, bipolare Störung oder Hypercholesterolämie ist.

**7.** Verfahren nach einem der vorangehenden Ansprüche, wobei das Genexpressionsprofil das Genexpressionsprofil eines oder mehrerer Gene umfasst, die ausgewählt sind aus der Gruppe bestehend aus jenen Genen, die in Fig. 1A-1C aufgelistet sind.

**8.** Verfahren nach Anspruch 7, wobei das Genexpressionsprofil das Genexpressionsprofil eines oder mehrerer Gene umfasst, die ausgewählt sind aus der Gruppe bestehend aus: NEIL2, BLK, MSRA, C80RF13, PPP1R3B, TNKS, CTSB, ANGPT, TDH und C80RF7.

**9.** Verfahren nach einem der vorangehenden Ansprüche, wobei das Genexpressionsprofil unter Verwendung eines Hybridisierungstests an Oligonukleotide erhalten wird, die in einer Mikroarray enthalten sind.

**10.** Verfahren nach einem der vorangehenden Ansprüche, wobei das Genexpressionsprofil durch Nachweisen der Proteinprodukte der Gene erhalten wird.

**11.** Verfahren nach Anspruch 10, wobei Antikörper, die zu einer spezifischen Bindung von Proteinprodukten der Gene imstande sind, zum Nachweisen der Proteinprodukte der Gene verwendet werden.

**12.** Verfahren zum Vorhersagen der Wirksamkeit einer Arzneimittelbehandlung einer psychiatrischen Störung bei einer Person, wobei das Verfahren umfasst:

a) Erhalten des Genexpressionsprofils eines oder mehrerer informativer Gene von einer Probe, die von der zu behandelnden Person erhalten wird; und
b) Vergleichen des Genexpressionsprofils der Probe mit einem Referenzgenexpressionsprofil, wobei das Referenzprofil für eine spezifische Orientierung der genomischen Inv8p23-Region repräsentativ ist,

wobei ein Vergleich zwischen dem Probenexpressionsprofil und dem Referenzexpressionsprofil die Wirksamkeit der Arzneimittelbehandlung anzeigt.

**13.** Verfahren nach Anspruch 12, wobei die psychiatrische Störung eine Angststörung ist.

**14.** Verfahren nach Anspruch 13, wobei die Angststörung eine Panikstörung oder bipolare Störung ist.

**15.** Verfahren nach einem der Ansprüche 12 bis 14, wobei die psychiatrische Störung mit einer Störung komorbid ist, die ausgewählt ist aus der Gruppe bestehend aus: Depression, bipolarer Störung, Zwangsstörung, histrionischer Persönlichkeitsstörung, Familienentzug und Störung des familiären Gefüges, Hypercholesterolämie und Substanzenmissbrauch.

**16.** Verfahren zum Vorhersagen der Orientierung der genomischen Inv8p23-Region bei einer Person, wobei das Verfahren umfasst:

a) Erhalten eines Genexpressionsprofils eines oder mehrerer informativer Gene von einer Probe, die von einer zu diagnostizierenden Person erhalten wird; und
b) Vergleichen des Genexpressionsprofils der Probe mit einem Referenzgenexpressionsprofil, wobei das Referenzprofil für eine spezifische Orientierung der genomischen Inv8p23-Region repräsentativ ist,

wobei ein Vergleich zwischen dem Probenexpressionsprofil und dem Referenzexpressionsprofil die Orientierung anzeigt.

17. Verfahren nach Anspruch 16, wobei das Referenzprofil für die invertierte Orientierung der genomischen Inv8p23-Region repräsentativ ist.

18. Verfahren nach Anspruch 16-17, wobei das Genexpressionsprofil das Genexpressionsprofil eines oder mehrerer Gene umfasst, die ausgewählt sind aus der Gruppe bestehend aus jenen Genen, die in Fig. 1A-1C aufgelistet sind.

19. Verfahren nach Anspruch 16 bis 18, wobei das Genexpressionsprofil das Genexpressionsprofil eines oder mehrerer Gene umfasst, die ausgewählt sind aus der Gruppe bestehend aus: NEIL2, BLK, MSRA, C80RF13, PPP1R3B, TNKS, CTSB, ANGPT, TDH und C80RF7.

20. Verfahren nach einem der Ansprüche 16 bis 18, wobei eine invertierte Orientierung der genomischen Inv8p23-Region die Anfälligkeit für eine psychiatrische Störung oder komorbide Störung in Verbindung mit der psychiatrischen Störung anzeigt.

## Revendications

1. Procédé de diagnostic d'une susceptibilité à un trouble psychiatrique chez un individu, le procédé comprenant :

   a) l'obtention d'un profil d'expression génique d'un ou de plusieurs gènes informatifs d'un échantillon prélevé sur un individu à diagnostiquer ; et
   b) la comparaison du profil d'expression génique de l'échantillon à un profil d'expression génique de référence, le profil de référence étant représentatif d'une orientation spécifique de la région génomique d'Inv8p23,

   dans lequel la comparaison entre le profil d'expression de l'échantillon et le profil d'expression de référence indique la susceptibilité à un trouble psychiatrique.

2. Procédé selon la revendication 1, dans lequel le trouble psychiatrique est un trouble anxieux.

3. Procédé selon la revendication 2, dans lequel le trouble anxieux est la panique ou un trouble bipolaire.

4. Procédé selon l'une quelconque des revendications précédentes, dans lequel le profil de référence est représentatif de l'orientation inverse de la région génomique d'Inv8p23.

5. Procédé selon l'une quelconque des revendications précédentes, dans lequel le trouble psychiatrique est comorbide avec un trouble choisi dans le groupe constitué par : la dépression, un trouble bipolaire, un trouble obsessionnel compulsif, un trouble de la personnalité histrionique, les déni et dysfonctionnement familiaux, l'hypercholestérolémie et la toxicomanie.

6. Procédé selon la revendication 5, dans lequel le trouble comorbide est la dépression, un trouble bipolaire ou l'hypercholestérolémie.

7. Procédé selon l'une quelconque des revendications précédentes, dans lequel le profil d'expression génique comprend le profil d'expression génique d'un ou de plusieurs gènes choisis dans le groupe constitué par les gènes listés sur les figures 1A à 1C.

8. Procédé selon la revendication 7, dans lequel le profil d'expression génique comprend le profil d'expression génique d'un ou de plusieurs gènes choisis dans le groupe constitué par : NEIL2, BLK, MSRA, C80RF13, PPP1R3B, TNKS, CTSB, ANGPT, TDH et C80RF7.

9. Procédé selon l'une quelconque des revendications précédentes, dans lequel le profil d'expression génique est obtenu en utilisant un test d'hybridation aux oligonucléotides contenus dans une micro-puce.

**10.** Procédé selon l'une quelconque des revendications précédentes, dans lequel le profil d'expression génique est obtenu par détection des produits protéiques des gènes.

**11.** Procédé selon la revendication 10, dans lequel les anticorps capables de se lier spécifiquement aux produits protéiques des gènes sont utilisés pour détecter les produits protéiques des gènes.

**12.** Procédé de prédiction de l'efficacité d'un traitement médicamenteux d'un trouble psychiatrique chez un individu, le procédé comprenant :

a) l'obtention d'un profil d'expression génique d'un ou de plusieurs gènes informatifs d'un échantillon prélevé sur un individu à traiter ; et
b) la comparaison du profil d'expression génique de l'échantillon à un profil d'expression génique de référence, le profil de référence étant représentatif d'une orientation spécifique dans la région génomique d'Inv8p23,

dans lequel la comparaison entre le profil d'expression de l'échantillon et le profil d'expression de référence indique l'efficacité du traitement médicamenteux.

**13.** Procédé selon la revendication 12, dans lequel le trouble psychiatrique est un trouble anxieux.

**14.** Procédé selon la revendication 13, dans lequel le trouble anxieux est la panique ou un trouble bipolaire.

**15.** Procédé selon l'une quelconque des revendications 12 à 14, dans lequel le trouble psychiatrique est comorbide avec un trouble choisi dans le groupe constitué par : la dépression, un trouble bipolaire, un trouble obsessionnel compulsif, un trouble de la personnalité histrionique, les déni et dysfonctionnement familiaux, l'hypercholestérolémie et la toxicomanie.

**16.** Procédé de prédiction de l'orientation de la région génomique d'Inv8p23 chez un individu, le procédé comprenant :

a) l'obtention d'un profil d'expression génique d'un ou de plusieurs gènes informatifs d'un échantillon prélevé sur un individu à diagnostiquer ; et
b) la comparaison du profil d'expression génique de l'échantillon à un profil d'expression génique de référence, le profil de référence étant représentatif d'une orientation spécifique de la région génomique d'Inv8p23,

dans lequel la comparaison entre le profil d'expression de l'échantillon et le profil d'expression de référence indique ladite orientation.

**17.** Procédé selon la revendication 16, dans lequel le profil de référence est représentatif de l'orientation inverse de la région génomique d'Inv8p23.

**18.** Procédé selon les revendications 16 à 17, dans lequel le profil d'expression génique comprend le profil d'expression génique d'un ou de plusieurs gènes choisis dans le groupe constitué par les gènes listés sur les figures 1A à 1C.

**19.** Procédé selon les revendications 16 à 18, dans lequel le profil d'expression génique comprend le profil d'expression génique d'un ou de plusieurs gènes choisis dans le groupe constitué par : NEIL2, BLK, MSRA, C80RF13, PPP1R3B, TNKS, CTSB, ANGPT, TDH et C80RF7.

**20.** Procédé selon l'une quelconque des revendications 16 à 19, dans lequel une orientation inverse de la région génomique d'Inv8p23 indique la susceptibilité à un trouble psychiatrique ou à un trouble comorbide associé au trouble psychiatrique.

| Chromosome | Start | End | Ens Gene ID | HUGO ID |
|---|---|---|---|---|
| 8 | 6251530 | 6488550 | ENSG00000147316.1 | |
| 8 | 6347601 | 6408170 | ENSG00000091879.2 | ANGPT2 |
| 8 | 6461593 | 6462075 | ENSG00000177806.1 | |
| 8 | 6553596 | 6604592 | ENSG00000155189.2 | |
| 8 | 6656129 | 6670220 | ENSG00000186530.1 | |
| 8 | 6656129 | 6670220 | ENSG00000186530.1 | |
| 8 | 6715511 | 6722856 | ENSG00000164825.1 | DEFB1 |
| 8 | 6743897 | 6744400 | ENSG00000184519.3 | |
| 8 | 6769631 | 6771008 | ENSG00000164822.1 | DEFA6 |
| 8 | 6780755 | 6783196 | ENSG00000164821.1 | DEFA4 |
| 8 | 6822585 | 6825017 | ENSG00000184944.2 | DEFA1 |
| 8 | 6833023 | 6833184 | ENSG00000182391.1 | DEFT1 |
| 8 | 6841702 | 6844127 | ENSG00000173324.1 | DEFA1 |
| 8 | 6852126 | 6852287 | ENSG00000182110.1 | DEFT1 |
| 8 | 6860805 | 6863226 | ENSG00000185918.1 | DEFA3 |
| 8 | 6900239 | 6901669 | ENSG00000164816.3 | DEFA5 |
| 8 | 7091658 | 7093284 | ENSG00000177306.2 | |
| 8 | 7102419 | 7102787 | ENSG00000183257.1 | |
| 8 | 7110041 | 7110409 | ENSG00000182555.1 | |
| 8 | 7132907 | 7133275 | ENSG00000182099.1 | |
| 8 | 7177424 | 7183633 | ENSG00000184581.1 | |
| 8 | 7218076 | 7222432 | ENSG00000182131.1 | DEFB108 |
| 8 | 7259788 | 7261795 | ENSG00000177257.1 | DEFB4 |
| 8 | 7273828 | 7275280 | ENSG00000177243.1 | DEFB103 |
| 8 | 7292686 | 7308602 | ENSG00000164871.4 | SPAG11 |
| 8 | 7292686 | 7308602 | ENSG00000164871.4 | |
| 8 | 7292686 | 7308602 | ENSG00000164871.4 | |
| 8 | 7292686 | 7308602 | ENSG00000164871.4 | |
| 8 | 7315236 | 7320014 | ENSG00000177023.1 | DEFB104 |
| 8 | 7327436 | 7331319 | ENSG00000187082.1 | DEFB106 |
| 8 | 7332653 | 7334483 | ENSG00000186599.2 | DEFB105 |
| 8 | 7332653 | 7334483 | ENSG00000186599.2 | |
| 8 | 7436950 | 7437525 | ENSG00000186600.1 | |
| 8 | 7610958 | 7611326 | ENSG00000186550.1 | |
| 8 | 7618606 | 7618974 | ENSG00000186553.1 | |
| 8 | 7626254 | 7626622 | ENSG00000186555.1 | |
| 8 | 7633902 | 7634270 | ENSG00000186558.1 | |
| 8 | 7641549 | 7641917 | ENSG00000186560.1 | |
| 8 | 7649197 | 7649565 | ENSG00000186647.1 | |

FIG. 1A

| Chromosome | Start | End | Ens Gene ID | HUGO ID |
|---|---|---|---|---|
| 8 | 7706752 | 7710548 | ENSG00000186572.1 | DEFB107 |
| 8 | 7716940 | 7718770 | ENSG00000186562.2 | DEFB105 |
| 8 | 7716940 | 7718770 | ENSG00000186562.2 | |
| 8 | 7720104 | 7723985 | ENSG00000186579.1 | DEFB106 |
| 8 | 7731403 | 7736178 | ENSG00000176782.1 | DEFB104 |
| 8 | 7742812 | 7758728 | ENSG00000178287.4 | SPAG11 |
| 8 | 7742812 | 7758728 | ENSG00000178287.4 | |
| 8 | 7742812 | 7758728 | ENSG00000178287.4 | |
| 8 | 7742812 | 7758728 | ENSG00000178287.4 | |
| 8 | 7776136 | 7777588 | ENSG00000176797.1 | DEFB103 |
| 8 | 7789609 | 7791647 | ENSG00000171711.1 | DEFB4 |
| 8 | 7829344 | 7833702 | ENSG00000184866.1 | DEFB108 |
| 8 | 7866599 | 7872812 | ENSG00000184721.1 | |
| 8 | 7909333 | 7909701 | ENSG00000184376.1 | |
| 8 | 7934942 | 7935517 | ENSG00000129394.1 | |
| 8 | 8096209 | 8096802 | ENSG00000164862.1 | ERVK2 |
| 8 | 8123537 | 8132176 | ENSG00000164845.3 | |
| 8 | 8123537 | 8132176 | ENSG00000164845.3 | |
| 8 | 8135633 | 8136076 | ENSG00000173295.1 | |
| 8 | 8212676 | 8276599 | ENSG00000182319.1 | |
| 8 | 8692281 | 8787978 | ENSG00000147324.2 | MFHAS1 |
| 8 | 8897764 | 8928137 | ENSG00000104626.3 | |
| 8 | 8897764 | 8928137 | ENSG00000104626.3 | |
| 8 | 8955447 | 8956106 | ENSG00000129375.1 | |
| 8 | 9032916 | 9045616 | ENSG00000173281.1 | PPP1R3B |
| 8 | 9450855 | 9671801 | ENSG00000173273.2 | TNKS |
| 8 | 9949240 | 10323808 | ENSG00000175806.1 | MSRA |
| 8 | 10420466 | 10449086 | ENSG00000184647.1 | |
| 8 | 10501272 | 10550027 | ENSG00000183638.1 | RP1L1 |
| 8 | 10567581 | 10595509 | ENSG00000171060.3 | |
| 8 | 10618688 | 10625432 | ENSG00000171056.1 | SOX7 |
| 8 | 10660321 | 10729693 | ENSG00000104637.2 | |
| 8 | 10791073 | 10897403 | ENSG00000171044.1 | |
| 8 | 11003096 | 11003386 | ENSG00000178273.1 | C8orf5 |
| 8 | 11018707 | 11019012 | ENSG00000173619.1 | C8orf15 |
| 8 | 11023721 | 11023963 | ENSG00000178260.1 | |
| 8 | 11032417 | 11032701 | ENSG00000178254.1 | C8orf6 |
| 8 | 11089392 | 11096258 | ENSG00000164724.1 | C8orf7 |
| 8 | 11142546 | 11142632 | ENSG00000171029.1 | C8orf8 |
| 8 | 11178927 | 11179289 | ENSG00000178223.1 | |
| 8 | 11179410 | 11223062 | ENSG00000104643.1 | MTMR9 |

FIG. 1B

| Chromosome | Start | End | Ens Gene ID | HUGO ID |
|---|---|---|---|---|
| 8 | 11226026 | 11227042 | ENSG00000164729.1 | AMAC |
| 8 | 11242564 | 11263371 | ENSG00000154316.3 | TDH |
| 8 | 11242564 | 11263371 | ENSG00000154316.3 | |
| 8 | 11316382 | 11361663 | ENSG00000154319.1 | C8orf13 |
| 8 | 11329985 | 11333461 | ENSG00000184608.1 | C8orf12 |
| 8 | 11388930 | 11459516 | ENSG00000136573.1 | BLK |
| 8 | 11471453 | 11476259 | ENSG00000170983.1 | C8orf14 |
| 8 | 11599162 | 11654918 | ENSG00000136574.4 | GATA4 |
| 8 | 11599162 | 11654918 | ENSG00000136574.4 | |
| 8 | 11656174 | 11658141 | ENSG00000170976.1 | |
| 8 | 11664666 | 11682263 | ENSG00000154328.1 | NEIL2 |
| 8 | 11684923 | 11685306 | ENSG00000177907.1 | |
| 8 | 11697664 | 11733527 | ENSG00000079459.1 | FDFT1 |
| 8 | 11739236 | 11763044 | ENSG00000164733.4 | CTSB |
| 8 | 11739236 | 11763044 | ENSG00000164733.4 | CTSB |
| 8 | 11823849 | 11824424 | ENSG00000177586.2 | |
| 8 | 12027341 | 12033573 | ENSG00000182945.1 | |
| 8 | 12077763 | 12077915 | ENSG00000186534.1 | |
| 8 | 12080356 | 12088988 | ENSG00000186523.1 | |
| 8 | 12261159 | 12268240 | ENSG00000186658.1 | |
| 8 | 12293003 | 12293155 | ENSG00000184352.1 | |
| 8 | 12295594 | 12304218 | ENSG00000145002.2 | |
| 8 | 12431742 | 12434789 | ENSG00000185161.1 | |
| 8 | 12446181 | 12449164 | ENSG00000177405.1 | |
| 8 | 12462438 | 12464200 | ENSG00000183697.1 | |
| 8 | 12552144 | 12554889 | ENSG00000177400.2 | |
| 8 | 12552144 | 12554889 | ENSG00000177400.2 | |
| 8 | 12589785 | 12604647 | ENSG00000154359.1 | |
| 8 | 12819231 | 12875545 | ENSG00000177357.2 | |
| 8 | 12880139 | 12894040 | ENSG00000170941.2 | |
| 8 | 12951238 | 13382761 | ENSG00000164741.3 | DLC1 |
| 8 | 12951238 | 13382761 | ENSG00000164741.3 | |

FIG. 1C

Expression Results for CTSB, TDH, BLK, TNKS, C8ORF13

| INV CT | bActin 2^-ct | CTSB 2^-ct | TDH 2^-ct | BLK 2^-ct | TNKS 2^-ct | C8ORF13 2^-ct |
|---|---|---|---|---|---|---|
| 0 | 1,34272E-06 | 1,86707E-07 | 5,13422E-11 | 1,42415E-08 | 1,70605E-08 | 3,59166E-10 |
| 0 | 1,57241E-06 | 1,75634E-07 | 5,99355E-11 | 2,39504E-08 | 2,39958E-08 | 7,77368E-10 |
| 0 | 2,00803E-06 | 2,20172E-07 | 1,23819E-10 | 2,66455E-08 | 3,33498E-08 | 5,66549E-10 |
| 0 | 1,60018E-06 | 1,85321E-07 | 7,04408E-11 | 1,88877E-08 | 1,84805E-08 | 6,86789E-10 |
| 0 | 1,29066E-06 | 1,66801E-07 | 4,47131E-11 | 1,78575E-08 | 1,65712E-08 | 2,60713E-10 |
| 0 | 1,82845E-06 | 2,17481E-07 | 1,03918E-10 | 7,7503E-09 | 2,11292E-08 | 5,77948E-10 |
| 0 | 1,74195E-06 | 3,06882E-07 | 1,00751E-10 | 2,75451E-08 | 2,33909E-08 | 9,06888E-10 |
| 0 | 1,5592E-06 | 1,87459E-07 | 8,78927E-11 | 2,12164E-08 | 2,0085E-08 | 5,78846E-10 |
| 0 | 1,96399E-06 | 2,76854E-07 | 1,14251E-10 | 1,41807E-08 | 2,57233E-08 | 7,50133E-10 |
| 0 | 1,1988E-06 | 1,04414E-07 | | 1,00352E-08 | 1,78545E-08 | 2,52012E-10 |
| 0 | 9,96811E-07 | 1,58308E-07 | 4,08845E-11 | 1,58125E-08 | 1,37458E-08 | 3,86636E-10 |
| 0 | 1,03097E-06 | 1,14701E-07 | 5,20945E-11 | 6,75044E-09 | 1,29809E-08 | 3,94808E-10 |
| 0 | 1,07543E-06 | 1,78154E-07 | 4,4412E-11 | 4,95258E-08 | 1,68435E-08 | 8,35725E-10 |
| 0 | 2,97408E-06 | 3,14858E-07 | 1,41887E-10 | 3,93234E-08 | 4,96185E-08 | 7,627E-10 |
| 0 | 3,22124E-06 | 4,79994E-07 | 9,17654E-11 | 3,55358E-08 | 4,15396E-08 | 9,81282E-10 |
| 0 | 1,10658E-06 | 1,33255E-07 | 6,45289E-11 | 1,84669E-08 | 2,23882E-08 | 4,52468E-10 |
| 0 | 4,57693E-06 | 5,07439E-07 | 1,65988E-10 | 3,45793E-08 | 4,96E-08 | 5,78614E-10 |
| 0 | 8,76485E-07 | 1,51311E-07 | | 7,63798E-09 | 1,2868E-08 | 1,84914E-10 |
| 0 | 1,48158E-06 | 2,24649E-07 | | 1,22339E-08 | 1,99936E-08 | 3,45697E-10 |
| 0 | 2,31148E-06 | 3,46506E-07 | 1,29701E-10 | 2,9605E-08 | 3,03282E-08 | 8,48325E-10 |
| 0 | 1,63373E-06 | 2,12639E-07 | · | 3,69389E-08 | 2,54179E-08 | 6,85718E-10 |
| 0 | 3,8221E-06 | 5,39107E-07 | 2,05261E-10 | 9,59373E-08 | 6,01621E-08 | 1,64435E-09 |
| 0 | 1,12097E-06 | 1,51644E-07 | | 9,73274E-09 | 1,25204E-08 | 3,49846E-10 |
| 0 | 4,93708E-07 | 7,67075E-08 | | 6,56649E-09 | 7,88425E-09 | 1,89268E-10 |
| 0 | 1,24267E-06 | 1,64109E-07 | 5,83413E-11 | 6,26594E-09 | 1,80549E-08 | 2,15268E-10 |
| 0 | 4,14866E-07 | 6,91077E-08 | | 5,14343E-09 | 1,04505E-08 | 3,49023E-10 |
| 0 | 1,98141E-06 | 3,17008E-07 | 1,3465E-10 | 9,05259E-09 | 2,64704E-08 | 6,97974E-10 |
| 0 | 1,18937E-06 | 1,70297E-07 | 6,41899E-11 | 2,16572E-08 | 2,23693E-08 | 4,95215E-10 |
| 0 | 1,78583E-06 | 2,4704E-07 | 3,49576E-11 | 1,87706E-08 | 2,39702E-08 | 6,94668E-10 |
| 0 | 1,30745E-06 | 1,87582E-07 | 8,20376E-11 | 2,43053E-08 | 2,1682E-08 | 6,55282E-10 |
| 0 | 1,10435E-06 | 1,12374E-07 | | 1,25746E-08 | 1,59022E-08 | 3,94292E-10 |
| 1 | 1,41259E-06 | 1,76734E-07 | 8,34477E-11 | 1,11265E-08 | 2,19361E-08 | 3,75769E-10 |
| 1 | 1,14281E-06 | 1,29305E-07 | 2,66105E-11 | 1,35412E-08 | 1,5627E-08 | 1,13797E-09 |
| 1 | 1,1806E-06 | 1,7238E-07 | 6,58869E-11 | 2,55548E-08 | 2,11336E-08 | 1,33862E-09 |
| 1 | 3,14462E-06 | 3,68594E-07 | 1,20246E-10 | 2,5124E-08 | 3,9357E-08 | 2,18792E-09 |
| 1 | 1,3848E-06 | 1,86246E-07 | 4,86503E-11 | 7,51157E-09 | 2,08911E-08 | 1,51698E-10 |
| 1 | 3,94542E-06 | 3,9269E-07 | | 4,92635E-08 | 4,76684E-08 | 9,13896E-10 |
| 1 | 2,43374E-06 | 2,35948E-07 | 1,43644E-10 | 2,75103E-08 | 3,46911E-08 | 2,18448E-09 |
| 1 | 1,09972E-06 | 1,65207E-07 | | 8,51007E-09 | 1,46193E-08 | 4,43843E-10 |
| 1 | 1,53081E-06 | 1,53912E-07 | 3,76551E-11 | 8,25088E-09 | 1,31052E-08 | 5,40574E-10 |
| 1 | 6,60034E-07 | 8,96711E-08 | 6,60207E-12 | 6,28682E-09 | 9,50818E-09 | 4,80076E-10 |
| 1 | 8,04023E-07 | 1,10108E-07 | | 4,26121E-09 | 1,16174E-08 | 4,0765E-10 |
| 1 | 1,53664E-06 | 1,47236E-07 | | 1,72157E-08 | 2,12031E-08 | 1,0901E-09 |
| 1 | 1,2275E-06 | 1,31984E-07 | | 1,26293E-08 | 1,477E-08 | 3,47907E-10 |
| 1 | 1,47533E-06 | 1,49887E-07 | 5,08165E-11 | 3,05898E-08 | 2,11063E-08 | 3,16144E-10 |
| 1 | 1,87272E-06 | 1,93602E-07 | 2,89944E-11 | 1,85634E-08 | 2,33861E-08 | 1,1766E-09 |
| 1 | 1,31609E-06 | 2,10622E-07 | | 1,03586E-08 | 1,49926E-08 | 6,22105E-10 |
| 1 | 1,45309E-06 | 2,1616E-07 | 4,54608E-11 | 1,69724E-08 | 1,56671E-08 | 4,22682E-10 |

FIG. 2A

Expression Results for CTSB, TDH, BLK, TNKS, C8ORF13

| INV CT | bActin 2^-ct | CTSB 2^-ct | TDH 2^-ct | BLK 2^-ct | TNKS 2^-ct | C8ORF13 2^-ct |
|---|---|---|---|---|---|---|
| 1 | 1,80235E-06 | 2,11714E-07 | 8,68183E-11 | 1,74673E-08 | 2,34835E-08 | 4,78391E-10 |
| 1 | 1,81092E-06 | 2,21327E-07 | | 2,98575E-08 | 2,53319E-08 | 1,20805E-09 |
| 1 | 2,70175E-06 | 3,97871E-07 | | 2,83628E-08 | 3,53929E-08 | 1,06269E-09 |
| 1 | 8,65051E-07 | 8,61127E-08 | | 1,22333E-08 | 1,41775E-08 | 1,08686E-09 |
| 1 | 8,85299E-07 | 1,34033E-07 | 2,34013E-11 | 1,07723E-08 | 1,25269E-08 | 5,07598E-10 |
| 1 | 1,29534E-06 | 1,46505E-07 | 1,97507E-11 | 9,59889E-09 | 1,53886E-08 | 8,40853E-10 |
| 1 | 1,54604E-06 | 1,65417E-07 | 4,00998E-11 | 2,12525E-08 | 2,45361E-08 | 5,33453E-10 |
| 1 | 1,21085E-06 | 1,46551E-07 | | 6,54643E-09 | 1,45911E-08 | 7,45751E-10 |
| 1 | 9,76411E-07 | 1,40604E-07 | 2,7853E-11 | 6,60416E-09 | 1,46435E-08 | 4,86776E-10 |
| 1 | 3,59978E-06 | 4,11771E-07 | | 2,435E-08 | 3,37028E-08 | 3,09537E-09 |
| 1 | 1,55392E-06 | 1,11436E-07 | 5,0995E-11 | 1,36442E-08 | 1,86372E-08 | 2,3862E-10 |
| 1 | 1,86262E-06 | 2,99798E-07 | | 1,49446E-08 | 2,38679E-08 | 7,81711E-10 |
| 1 | 1,43542E-06 | 1,75252E-07 | | 1,51584E-08 | 2,50776E-08 | 5,84978E-10 |
| 1 | 1,33616E-06 | 1,79154E-07 | 5,98822E-11 | 1,36883E-08 | 1,80634E-08 | 8,73799E-10 |
| 1 | 1,15751E-06 | 1,43457E-07 | 3,29692E-11 | 5,28033E-09 | 1,53139E-08 | 7,63748E-10 |
| 2 | 1,72501E-06 | 1,59373E-07 | 1,24423E-11 | 4,82684E-09 | 1,43438E-08 | 7,91594E-10 |
| 2 | 1,73259E-06 | 2,2109E-07 | 3,61263E-11 | 8,99329E-09 | 2,00252E-08 | 1,68788E-09 |
| 2 | 1,65752E-06 | 2,01916E-07 | | 1,57154E-08 | 2,06741E-08 | 1,34333E-09 |
| 2 | 1,50989E-06 | 2,19221E-07 | 7,16274E-11 | 1,38693E-08 | 1,90122E-08 | 4,91335E-10 |
| 2 | 1,59413E-06 | 1,72794E-07 | | 1,54756E-08 | 2,04271E-08 | 2,53171E-09 |
| 2 | 1,43159E-06 | 2,04548E-07 | | 1,49013E-08 | 1,91665E-08 | 2,12827E-09 |
| 2 | 1,51218E-06 | 1,32536E-07 | 4,24793E-11 | 5,02471E-08 | 1,96175E-08 | 5,27718E-10 |
| 2 | 3,80033E-06 | 4,27536E-07 | 2,02344E-10 | 2,80254E-08 | 6,06604E-08 | 2,38544E-09 |
| 2 | 1,16012E-06 | 1,24284E-07 | 4,85939E-11 | 8,07041E-09 | 1,47652E-08 | 1,1298E-09 |
| 2 | 2,5777E-06 | 2,76102E-07 | 1,01791E-10 | 3,20052E-08 | 2,89578E-08 | 2,37015E-09 |
| 2 | 1,43554E-06 | 1,50128E-07 | 4,90076E-11 | 1,11705E-08 | 1,70564E-08 | 1,2701E-09 |
| 2 | 2,34837E-06 | 2,66974E-07 | 6,59268E-11 | 1,41144E-08 | 2,31578E-08 | 1,71325E-09 |
| 2 | 8,96786E-07 | 1,09802E-07 | 4,96864E-11 | 1,47185E-08 | 1,47755E-08 | 8,4611E-10 |
| 2 | 1,21109E-06 | 1,47878E-07 | 2,98208E-11 | 2,53982E-08 | 1,81213E-08 | 1,88072E-09 |
| 2 | 1,1706E-06 | 1,23502E-07 | | 7,43764E-09 | 1,45637E-08 | 1,50112E-09 |
| 2 | 3,86486E-06 | 4,86115E-07 | 9,39743E-11 | 2,94536E-08 | 5,1379E-08 | 2,4662E-09 |
| 2 | 1,59987E-06 | 2,23263E-07 | 3,40048E-11 | 1,39991E-08 | 2,36124E-08 | 1,36872E-09 |
| 2 | 1,20598E-06 | 1,32338E-07 | 1,66667E-11 | 1,6603E-08 | 1,54482E-08 | 1,30887E-09 |
| 2 | 1,53676E-06 | 1,32203E-07 | | 1,78062E-08 | 1,62514E-08 | 4,5317E-10 |
| 2 | 9,57786E-07 | 8,9325E-08 | | 8,85579E-09 | 1,20897E-08 | 1,50074E-09 |
| 2 | 1,0226E-06 | 9,6144E-08 | 4,40068E-11 | 7,01441E-09 | 1,24703E-08 | 3,09131E-10 |
| 2 | 4,28729E-06 | 5,28639E-07 | 1,24572E-10 | 4,20238E-08 | 4,44843E-08 | 1,2185E-09 |
| 2 | 1,59847E-06 | 1,61866E-07 | 4,09895E-11 | 2,33004E-08 | 2,3848E-08 | 6,51306E-10 |
| 2 | 1,12605E-06 | 1,35177E-07 | 3,6561E-11 | 1,53575E-08 | 1,55848E-08 | 2,08548E-09 |
| 2 | 7,89479E-07 | 8,52628E-08 | | 6,1465E-09 | 1,23459E-08 | 9,49881E-10 |
| 2 | 8,29308E-07 | 6,50752E-08 | 1,70014E-11 | 7,63902E-09 | 1,48856E-08 | 1,55559E-10 |
| 2 | 1,1537E-06 | 1,81156E-07 | | 2,29947E-08 | 2,29621E-08 | 1,56379E-09 |
| 2 | 9,47826E-07 | 1,18308E-07 | 1,93321E-11 | 7,62968E-09 | 1,33612E-08 | 1,66731E-09 |
| 2 | 8,92082E-07 | 1,24002E-07 | 4,19293E-11 | 1,60127E-08 | 1,64535E-08 | 9,32505E-10 |
| 2 | 2,70878E-06 | 3,3161E-07 | 7,4006E-11 | 3,34708E-08 | 3,62791E-08 | 2,84215E-09 |
| 2 | 5,89248E-07 | 7,91378E-08 | | 1,03815E-08 | 2,24806E-08 | 1,99944E-09 |
| 2 | 1,3056E-06 | 1,25777E-07 | 4,20923E-11 | 1,09249E-08 | 1,75496E-08 | 3,42258E-10 |

FIG. 2B

Relative Expression

| INV CT | CTSB/bActin | TDH/bActin | BLK/bActin | TNKS/bActin | C8ORF13/bActin |
|---|---|---|---|---|---|
| 0 | 0,139051455 | 3,82375E-05 | 0,010606462 | 0,012705947 | 0,000267492 |
| 0 | 0,111697535 | 3,8117E-05 | 0,015231663 | 0,015260571 | 0,000494381 |
| 0 | 0,109645689 | 6,16617E-05 | 0,013269428 | 0,016608194 | 0,000282141 |
| 0 | 0,115812148 | 4,40205E-05 | 0,011803434 | 0,011548978 | 0,000429194 |
| 0 | 0,129237419 | 3,46437E-05 | 0,013835957 | 0,01283937 | 0,000202 |
| 0 | 0,118943101 | 5,68342E-05 | 0,004238735 | 0,011555824 | 0,000316087 |
| 0 | 0,176171648 | 5,78378E-05 | 0,015812827 | 0,013427995 | 0,000520617 |
| 0 | 0,120227931 | 5,63703E-05 | 0,013607228 | 0,012881603 | 0,000371246 |
| 0 | 0,140964765 | 5,8173E-05 | 0,007220318 | 0,013097448 | 0,000381943 |
| 0 | 0,087099108 | | 0,008371 | 0,014893599 | 0,00021022 |
| 0 | 0,158814991 | 4,10153E-05 | 0,015863122 | 0,013789787 | 0,000387873 |
| 0 | 0,111255302 | 5,05294E-05 | 0,00654763 | 0,012590946 | 0,000382947 |
| 0 | 0,165658714 | 4,12969E-05 | 0,04605206 | 0,015662106 | 0,000777107 |
| 0 | 0,105867304 | 4,77079E-05 | 0,013222039 | 0,016683647 | 0,000256449 |
| 0 | 0,149008884 | 2,84876E-05 | 0,011031695 | 0,012895515 | 0,000304628 |
| 0 | 0,120420465 | 5,83137E-05 | 0,016688244 | 0,0202318 | 0,000408888 |
| 0 | 0,110868986 | 3,62662E-05 | 0,007555127 | 0,010836959 | 0,00012642 |
| 0 | 0,172633457 | | 0,008714333 | 0,014681412 | 0,000210972 |
| 0 | 0,151627832 | | 0,008257338 | 0,01349474 | 0,00023333 |
| 0 | 0,14990623 | 5,61117E-05 | 0,01280778 | 0,013120666 | 0,000367004 |
| 0 | 0,130155438 | | 0,022610207 | 0,0155582 | 0,000419726 |
| 0 | 0,141049895 | 5,37036E-05 | 0,025100649 | 0,015740564 | 0,000430222 |
| 0 | 0,135279276 | | 0,008682431 | 0,011169263 | 0,000312092 |
| 0 | 0,155370176 | | 0,013300336 | 0,015969451 | 0,00038336 |
| 0 | 0,132061812 | 4,69484E-05 | 0,005042323 | 0,014529151 | 0,00017323 |
| 0 | 0,166578401 | | 0,012397807 | 0,025189953 | 0,000841291 |
| 0 | 0,159991371 | 6,79567E-05 | 0,004568768 | 0,013359409 | 0,000352262 |
| 0 | 0,143182666 | 5,39697E-05 | 0,018208953 | 0,018807674 | 0,000416368 |
| 0 | 0,138333886 | 1,9575E-05 | 0,010510885 | 0,013422479 | 0,00038899 |
| 0 | 0,143471616 | 6,27461E-05 | 0,018589796 | 0,016583407 | 0,00050119 |
| 0 | 0,101756281 | | 0,011386469 | 0,014399677 | 0,000357037 |
| 1 | 0,125113186 | 5,90741E-05 | 0,007876619 | 0,015528919 | 0,000266013 |
| 1 | 0,113146138 | 2,32851E-05 | 0,011849022 | 0,013674167 | 0,000995766 |
| 1 | 0,146010295 | 5,58078E-05 | 0,021645508 | 0,01790064 | 0,001133841 |
| 1 | 0,117213976 | 3,82385E-05 | 0,0079895 | 0,012515632 | 0,000695764 |
| 1 | 0,134492744 | 3,51316E-05 | 0,005424285 | 0,015085944 | 0,000109544 |
| 1 | 0,099530448 | | 0,012486247 | 0,012081947 | 0,000231635 |
| 1 | 0,096948592 | 5,9022E-05 | 0,011303734 | 0,014254243 | 0,000897582 |
| 1 | 0,150226658 | | 0,007738396 | 0,013293619 | 0,000403596 |
| 1 | 0,100543275 | 2,45982E-05 | 0,005389886 | 0,008560955 | 0,00035313 |
| 1 | 0,135858313 | 1,00026E-05 | 0,009524993 | 0,014405592 | 0,000727351 |
| 1 | 0,136945783 | | 0,005299861 | 0,014449138 | 0,000507013 |
| 1 | 0,095816937 | | 0,011203509 | 0,013798383 | 0,000709405 |
| 1 | 0,107522202 | | 0,010288668 | 0,012032561 | 0,000283427 |
| 1 | 0,101595947 | 3,44442E-05 | 0,02073423 | 0,014306184 | 0,000214288 |
| 1 | 0,103380017 | 1,54825E-05 | 0,009912521 | 0,012487732 | 0,000628283 |
| 1 | 0,16003618 | | 0,00787074 | 0,011391776 | 0,000472691 |
| 1 | 0,148758808 | 3,12856E-05 | 0,011680206 | 0,010781932 | 0,000290885 |

FIG. 2C

Relative Expression

| INV CT | CTSB/bActin | TDH/bActin | BLK/bActin | TNKS/bActin | C8ORF13/bActin |
|---|---|---|---|---|---|
| 1 | 0,117465459 | 4,81696E-05 | 0,009691442 | 0,013029396 | 0,000265426 |
| 1 | 0,122217773 | | 0,016487466 | 0,013988383 | 0,000667089 |
| 1 | 0,147263801 | | 0,010497919 | 0,013099988 | 0,000393332 |
| 1 | 0,09954642 | | 0,014141767 | 0,016389263 | 0,001256408 |
| 1 | 0,151399018 | 2,64332E-05 | 0,012167936 | 0,014149852 | 0,000573364 |
| 1 | 0,113101404 | 1,52475E-05 | 0,007410331 | 0,011879951 | 0,000649137 |
| 1 | 0,106993922 | 2,59371E-05 | 0,013746377 | 0,015870295 | 0,000345044 |
| 1 | 0,121031732 | | 0,00540647 | 0,01205032 | 0,00061589 |
| 1 | 0,144001201 | 2,85258E-05 | 0,006763706 | 0,014997312 | 0,000498536 |
| 1 | 0,114387859 | | 0,006764309 | 0,009362483 | 0,000859879 |
| 1 | 0,071712392 | 3,2817E-05 | 0,008780466 | 0,01199366 | 0,000153559 |
| 1 | 0,160954437 | | 0,008023421 | 0,012814134 | 0,000419683 |
| 1 | 0,122091316 | | 0,010560237 | 0,017470558 | 0,000407531 |
| 1 | 0,134081909 | 4,48168E-05 | 0,010244533 | 0,013518965 | 0,000653965 |
| 1 | 0,123935359 | 2,84828E-05 | 0,004561796 | 0,01323004 | 0,000659819 |
| 2 | 0,092389743 | 7,21291E-06 | 0,002798155 | 0,008315216 | 0,000458893 |
| 2 | 0,127606878 | 2,0851E-05 | 0,005190661 | 0,011557931 | 0,000974194 |
| 2 | 0,12181815 | | 0,009481303 | 0,012472932 | 0,000810449 |
| 2 | 0,145189699 | 4,74387E-05 | 0,009185593 | 0,012591775 | 0,000325411 |
| 2 | 0,108393752 | | 0,009707863 | 0,01281394 | 0,001588149 |
| 2 | 0,14288214 | | 0,010408965 | 0,01338829 | 0,001486654 |
| 2 | 0,087645586 | 2,80915E-05 | 0,033228334 | 0,012972994 | 0,000348979 |
| 2 | 0,112499773 | 5,32439E-05 | 0,007374477 | 0,015961892 | 0,000627693 |
| 2 | 0,10713071 | 4,1887E-05 | 0,006956544 | 0,012727351 | 0,000973866 |
| 2 | 0,107111776 | 3,9489E-05 | 0,012416204 | 0,011233976 | 0,000919484 |
| 2 | 0,104580063 | 3,41389E-05 | 0,007781447 | 0,011881541 | 0,000884759 |
| 2 | 0,113684542 | 2,80734E-05 | 0,006010301 | 0,009861228 | 0,000729546 |
| 2 | 0,122439107 | 5,5405E-05 | 0,01641254 | 0,016476086 | 0,000943491 |
| 2 | 0,122102424 | 2,4623E-05 | 0,020971258 | 0,014962777 | 0,001552909 |
| 2 | 0,105503243 | | 0,006353694 | 0,012441246 | 0,001282346 |
| 2 | 0,125778261 | 2,43151E-05 | 0,007620865 | 0,013293898 | 0,000638108 |
| 2 | 0,139550892 | 2,12548E-05 | 0,008750158 | 0,014758974 | 0,00085552 |
| 2 | 0,109735502 | 1,38201E-05 | 0,013767322 | 0,012809689 | 0,001085318 |
| 2 | 0,086027084 | | 0,011586862 | 0,010575134 | 0,000294887 |
| 2 | 0,093261902 | | 0,0092461 | 0,012622593 | 0,001566889 |
| 2 | 0,094018795 | 4,30341E-05 | 0,006859364 | 0,012194619 | 0,000302298 |
| 2 | 0,123303743 | 2,90561E-05 | 0,009801964 | 0,010375862 | 0,000284213 |
| 2 | 0,10126319 | 2,5643E-05 | 0,014576753 | 0,014919322 | 0,000407457 |
| 2 | 0,120045086 | 3,24684E-05 | 0,013638419 | 0,013840249 | 0,001852034 |
| 2 | 0,107998749 | | 0,007785514 | 0,015638067 | 0,001203174 |
| 2 | 0,078469265 | 2,05007E-05 | 0,009211313 | 0,017949451 | 0,000187577 |
| 2 | 0,157021444 | | 0,019931236 | 0,019902921 | 0,001355456 |
| 2 | 0,124820041 | 2,03963E-05 | 0,008049665 | 0,014096629 | 0,001759088 |
| 2 | 0,139002887 | 4,70016E-05 | 0,01794979 | 0,018443922 | 0,001045313 |
| 2 | 0,122420473 | 2,73208E-05 | 0,012356417 | 0,013393174 | 0,001049237 |
| 2 | 0,134303192 | | 0,017618208 | 0,038151392 | 0,003393214 |
| 2 | 0,096336429 | 3,22397E-05 | 0,008367723 | 0,013441759 | 0,000262145 |

| | PAERSON CORR |
|---|---|
| CTSB/βActin | -0,371115357 |
| TDH/bActin | -0,486183625 |
| BLK/bActin | -0,128733448 |
| TNKS/bActin | -0,042432854 |
| C8ORF13/bActin | -0,512108631 |

FIG. 2D

SUMMARY OUTPUT FOR CTSB/bActin

| Regression Statistics | |
|---|---|
| Multiple R | 0,371115357 |
| R Square | 0,137726608 |
| Adjusted R Square | 0,128454851 |
| Standard Error | 0,020985238 |
| Observations | 95 |

ANOVA

| | df | SS | MS | F | Significance F |
|---|---|---|---|---|---|
| Regression | 1 | 0,006541594 | 0,006541594 | 14,85442398 | 0,000213539 |
| Residual | 93 | 0,040955359 | 0,00044038 | | |
| Total | 94 | 0,047496953 | | | |

| | Coefficients | Standard Error | t Stat | P-value | Lower 95% | Upper 95% | Lower 95.0% | Upper 95.0% |
|---|---|---|---|---|---|---|---|---|
| Intercept | 0,134401237 | 0,003431453 | 39,16743887 | 1,35792E-59 | 0,127587058 | 0,141215417 | 0,127587058 | 0,141215417 |
| X Variable 1 | -0,010190789 | 0,002644112 | -3,854143741 | 0,000213539 | -0,015441467 | -0,004940111 | -0,015441467 | -0,004940111 |

FIG. 2E

t-Test: Two-Sample Assuming Equal Variances
βActin alone

|  | Variable 1 | Variable 2 |
|---|---|---|
| Mean | 1,67272E-06 | 1,6306E-06 |
| Variance | 8,25519E-13 | 8,27545E-13 |
| Observations | 31 | 32 |
| Pooled Variance | 8,26549E-13 | |
| Hypothesized Mean Difference | 0 | |
| df | 61 | |
| t Stat | 0,183868216 | |
| P(T<=t) one-tail | 0,427363346 | |
| t Critical one-tail | 1,670218808 | |
| P(T<=t) two-tail | 0,854726693 | |
| t Critical two-tail | 1,999624146 | |

t-Test: Two-Sample Assuming Equal Variances
CTSB/βActin

|  | Variable 1 | Variable 2 |
|---|---|---|
| Mean | 0,135230445 | 0,114822954 |
| Variance | 0,000509647 | 0,000355254 |
| Observations | 31 | 32 |
| Pooled Variance | 0,000431185 | |
| Hypothesized Mean Difference | 0 | |
| df | 61 | |
| t Stat | 3,899810317 | |
| P(T<=t) one-tail | 0,000121218 | |
| t Critical one-tail | 1,670218808 | |
| P(T<=t) two-tail | 0,000242436 | |
| t Critical two-tail | 1,999624146 | |

FIG. 2F

t-Test: Two-Sample Assuming Equal Variances
TDH/β–Actin

| | Variable 1 | Variable 2 |
|---|---|---|
| Mean | 8,64694E-11 | 5,63035E-11 |
| Variance | 2,17591E-21 | 1,80278E-21 |
| Observations | 24 | 23 |
| Pooled Variance | 1,99349E-21 | |
| Hypothesized Mean Difference | 0 | |
| df | 45 | |
| t Stat | 2,315412295 | |
| P(T<=t) one-tail | 0,012603268 | |
| t Critical one-tail | 1,679427442 | |
| P(T<=t) two-tail | 0,025206535 | |
| t Critical two-tail | 2,014103302 | |

t-Test: Two-Sample Assuming Equal Variances
BLK/β–Actin

| | Variable 1 | Variable 2 |
|---|---|---|
| Mean | 0,013262421 | 0,011293594 |
| Variance | 6,13485E-05 | 3,50261E-05 |
| Observations | 31 | 32 |
| Pooled Variance | 4,79715E-05 | |
| Hypothesized Mean Difference | 0 | |
| df | 61 | |
| t Stat | 1,127979739 | |
| P(T<=t) one-tail | 0,13187279 | |
| t Critical one-tail | 1,670218808 | |
| P(T<=t) two-tail | 0,263745581 | |
| t Critical two-tail | 1,999624146 | |

FIG. 2G

t-Test: Two-Sample Assuming Equal Variances
TNKS/β–Actin

|  | Variable 1 | Variable 2 |
|---|---|---|
| Mean | 0,014630204 | 0,014252088 |
| Variance | 8,37856E-06 | 2,50922E-05 |
| Observations | 31 | 32 |
| Pooled Variance | 1,68724E-05 |  |
| Hypothesized Mean Difference | 0 |  |
| df | 61 |  |
| t Stat | 0,365277021 |  |
| P(T<=t) one-tail | 0,358084079 |  |
| t Critical one-tail | 1,670218808 |  |
| P(T<=t) two-tail | 0,716168158 |  |
| t Critical two-tail | 1,999624146 |  |

t-Test: Two-Sample Assuming Equal Variances
C8ORF13/β–Actin

|  | Variable 1 | Variable 2 |
|---|---|---|
| Mean | 0,000371184 | 0,000982774 |
| Variance | 2,31843E-08 | 4,23758E-07 |
| Observations | 31 | 32 |
| Pooled Variance | 2,26755E-07 |  |
| Hypothesized Mean Difference | 0 |  |
| df | 61 |  |
| t Stat | -5,096447649 |  |
| P(T<=t) one-tail | 1,79957E-06 |  |
| t Critical one-tail | 1,670218808 |  |
| P(T<=t) two-tail | 3,59914E-06 |  |
| t Critical two-tail | 1,999624146 |  |

FIG. 2H

**Expression Results**

| INV CT | ANGPT-2 | OFR7 | 2^-ct MSRA | PPP1R3B | NEIL2 |
|---|---|---|---|---|---|
| 0 | 8,0368E-12 | 4,492E-11 | 1,276E-08 | 3,8273E-08 | 4,27463E-10 |
| 0 | | | 1,357E-08 | 2,23027E-08 | 8,90852E-10 |
| 0 | 1,6285E-11 | 4,9644E-11 | 2,503E-08 | 5,78805E-08 | 1,15651E-09 |
| 0 | | | 1,333E-08 | 3,12159E-08 | 8,39361E-10 |
| 0 | | | 1,078E-08 | 3,14805E-08 | 4,65533E-10 |
| 0 | 1,5871E-11 | 4,073E-11 | 1,582E-08 | 3,22324E-08 | 5,01473E-10 |
| 0 | | 4,3064E-11 | 1,879E-08 | 2,91476E-08 | 6,8144E-10 |
| 0 | 4,2472E-12 | 5,5859E-11 | 1,931E-08 | 3,14589E-08 | 1,29926E-09 |
| 0 | 9,839E-12 | | 1,988E-08 | 2,70495E-08 | 6,10744E-10 |
| 0 | | | 1,346E-08 | 3,42877E-08 | 6,34303E-10 |
| 0 | 6,4032E-12 | 2,3362E-11 | 9,457E-09 | 6,65089E-09 | 6,67662E-10 |
| 0 | | | 1,093E-08 | 8,30908E-09 | 5,05636E-10 |
| 0 | 8,0877E-12 | | 1,238E-08 | 1,68195E-08 | 5,10108E-10 |
| 0 | 1,5063E-11 | 8,389E-11 | 2,816E-08 | 4,62123E-08 | 2,07411E-09 |
| 0 | 1,6711E-11 | 1,9934E-11 | | 4,46306E-08 | 1,74817E-09 |
| 0 | 3,4623E-12 | 4,2657E-11 | 1,439E-08 | 1,50254E-08 | 8,49285E-10 |
| 0 | 6,1603E-11 | 6,6435E-11 | 7,092E-08 | 1,30571E-07 | 1,44568E-09 |
| 0 | | | 7,257E-09 | 1,03633E-08 | 3,72381E-10 |
| 0 | 6,7424E-12 | 2,2375E-11 | 1,329E-08 | 2,86475E-08 | 6,44684E-10 |
| 0 | 8,5478E-12 | 2,5563E-11 | 2,028E-08 | 4,85106E-08 | 1,67389E-09 |
| 0 | | 5,6873E-12 | 1,056E-08 | 9,84391E-10 | 1,11553E-10 |
| 0 | | | 1,409E-08 | 2,43937E-08 | 8,41288E-10 |
| 0 | | 1,2323E-10 | 3,012E-08 | 6,3345E-08 | 2,62422E-09 |
| 0 | | 2,0738E-11 | 8,279E-09 | 1,53166E-08 | 4,73891E-10 |
| 0 | | | 4,851E-09 | 3,06629E-09 | 3,93492E-10 |
| 0 | 5,2423E-12 | 4,1349E-11 | 1,167E-08 | 2,09546E-08 | 3,37236E-10 |
| 0 | 1,562E-12 | 1,2162E-11 | 6,526E-09 | 5,18032E-09 | 3,92002E-10 |
| 0 | | 6,655E-11 | 2,503E-08 | 2,74979E-08 | 1,08565E-09 |
| 0 | 3,371E-11 | | 1,537E-08 | 1,66305E-08 | 1,14711E-09 |
| 0 | | 3,1995E-11 | 2,316E-08 | 4,32827E-08 | 7,19927E-10 |
| 0 | 6,5097E-12 | 2,8147E-11 | 1,402E-08 | 2,06889E-08 | 8,80388E-10 |

**Relative Expression (ratio of gene expression to β-actin expression)**

| ANGPT-2 | C8ORF7 | MSRA | PPP1R3B | NEIL2 |
|---|---|---|---|---|
| 5,98545E-06 | 3,34547E-05 | 0,009506139 | 0,028504139 | 0,000318356 |
| | | 0,008628062 | 0,014183823 | 0,000566553 |
| 8,10989E-06 | 2,47225E-05 | 0,012463508 | 0,028824482 | 0,000575943 |
| | | 0,008327831 | 0,019507734 | 0,00052454 |
| | | 0,008348723 | 0,024391077 | 0,000360695 |
| 8,6799E-06 | 2,22756E-05 | 0,008654235 | 0,017628286 | 0,000274262 |
| | 2,47214E-05 | 0,010786149 | 0,016732719 | 0,000391194 |
| 2,72394E-06 | 3,58257E-05 | 0,012387261 | 0,020176294 | 0,000833285 |
| 5,00968E-06 | | 0,010123262 | 0,013772684 | 0,00031097 |
| | | 0,0112311814 | 0,0286011701 | 0,000529114 |
| 6,42372E-06 | 2,34369E-05 | 0,009486795 | 0,006672171 | 0,0006669798 |
| | | 0,010604861 | 0,008059441 | 0,000490445 |
| 7,52046E-06 | | 0,011514452 | 0,015639791 | 0,000474329 |
| 5,0647E-06 | 2,82071E-05 | 0,009467124 | 0,015538349 | 0,000697395 |
| 5,18784E-06 | 6,18845E-06 | | 0,013855074 | 0,000542702 |
| 3,12883E-06 | 3,85488E-05 | 0,01299964 | 0,013578242 | 0,0007767484 |
| 1,34596E-05 | 1,45152E-05 | 0,015494562 | 0,028528175 | 0,000315863 |
| | | 0,008279435 | 0,011823742 | 0,000424857 |
| 4,55081E-06 | 1,51021E-05 | 0,008972803 | 0,019335743 | 0,000435133 |
| 3,69797E-06 | 1,10591E-05 | 0,008774373 | 0,020986782 | 0,000724161 |
| | | | | |
| | | 0,008622515 | 0,014931325 | 0,00051495 |
| | 3,22403E-05 | 0,007879845 | 0,016573344 | 0,000686591 |
| | 1,85E-05 | 0,007385679 | 0,013663754 | 0,000422751 |
| | | 0,009826177 | 0,006210738 | 0,000797013 |
| 4,21855E-06 | 3,3274E-05 | 0,00939428 | 0,016862611 | 0,000271381 |
| 3,76511E-06 | 2,93157E-05 | 0,015730526 | 0,012486732 | 0,000944888 |
| 0 | 3,35873E-05 | 0,012633586 | 0,013877986 | 0,00054792 |
| 2,83427E-05 | | 0,012920026 | 0,013982661 | 0,000964471 |
| | 1,79159E-05 | 0,012969148 | 0,024236791 | 0,000403134 |
| 4,97891E-06 | 2,15279E-05 | 0,010724642 | 0,015823804 | 0,000673361 |

FIG. 3A

27

| | | | | | | | | | | |
|---|---|---|---|---|---|---|---|---|---|---|
| 0 | | | 1,384E-08 | 1,61702E-08 | 6,24187E-10 | | | 0,01253139 | 0,014642369 | 0,00056521 |
| 1 | | 4,9517E-11 | 1,477E-08 | 1,12718E-08 | 8,94396E-10 | | 3,50536E-05 | 0,010457913 | 0,007979473 | 0,000633159 |
| 1 | 7,115E-12 | 2,5617E-11 | 1,081E-08 | 8,00538E-09 | 8,30058E-10 | 6,22585E-06 | 2,24155E-05 | 0,009460848 | 0,007004981 | 0,000726329 |
| 1 | | | 1,208E-08 | 7,06199E-09 | 1,01668E-09 | | | 0,010233004 | 0,005981676 | 0,000861148 |
| 1 | | | 4,402E-08 | 3,62243E-08 | 1,67397E-09 | | | 0,013998024 | 0,011519441 | 0,000532328 |
| 1 | 2,1654E-11 | 5,873E-11 | 1,581E-08 | 1,54706E-08 | 1,33511E-09 | 1,56367E-05 | 4,24102E-05 | 0,011413517 | 0,011171709 | 0,000964115 |
| 1 | 3,7996E-11 | 7,4119E-11 | 2,537E-08 | 2,28462E-08 | 3,02982E-09 | 9,63037E-06 | 1,87862E-05 | 0,006429939 | 0,005790549 | 0,000767934 |
| 1 | 1,0855E-11 | 1,0548E-10 | 2,438E-08 | 3,90312E-08 | 2,14567E-09 | 4,46005E-06 | 4,33419E-05 | 0,010018683 | 0,016037535 | 0,000881637 |
| 1 | | 2,638E-11 | 1,412E-08 | 1,07011E-08 | 8,24463E-10 | | 2,39879E-05 | 0,012843091 | 0,009730716 | 0,000749702 |
| 1 | 3,7622E-12 | | 1,084E-08 | 1,3732E-08 | 9,39527E-10 | 2,45765E-06 | | 0,007079477 | 0,0089704 | 0,000613746 |
| 1 | 8,4864E-12 | 2,0552E-11 | 6,006E-09 | 6,24059E-09 | 5,08977E-10 | 1,28575E-05 | 3,11383E-05 | 0,009099338 | 0,009454951 | 0,000771138 |
| 1 | | | 1,024E-08 | 7,93344E-09 | 3,98956E-10 | | | 0,012737277 | 0,009867181 | 0,0004962 |
| 1 | | | 1,246E-08 | 1,0939E-08 | 1,07069E-09 | | | 0,008109142 | 0,007118789 | 0,000696773 |
| 1 | 1,5752E-11 | 4,1037E-11 | 1,092E-08 | 7,52477E-09 | 7,66649E-10 | 1,28329E-05 | 3,34316E-05 | 0,00889563 | 0,006130156 | 0,000624561 |
| 1 | | | 1,19E-08 | 1,27767E-08 | 1,72699E-09 | | | 0,008067977 | 0,008660267 | 0,001170584 |
| 1 | | 3,4763E-11 | 1,474E-08 | 1,55668E-08 | 1,32566E-09 | | 1,85626E-05 | 0,007872452 | 0,00831238 | 0,00070788 |
| 1 | | 2,5166E-11 | 9,382E-09 | 1,33293E-08 | 8,10956E-10 | | 1,91219E-05 | 0,007128696 | 0,01012795 | 0,000616185 |
| 1 | | 4,0912E-11 | 9,997E-09 | 1,13611E-08 | 5,49914E-10 | | 2,81554E-05 | 0,006880015 | 0,007818551 | 0,000378444 |
| 1 | | 6,7653E-11 | 1,272E-08 | 3,46594E-08 | 1,24698E-09 | | 3,7536E-05 | 0,007056285 | 0,019230139 | 0,000691865 |
| 1 | | 7,483E-11 | 1,8E-08 | 3,03994E-08 | | | 4,13217E-05 | 0,009939917 | 0,016786706 | |
| 1 | 3,5991E-12 | 1,2567E-10 | 2,595E-08 | 1,75379E-08 | 2,0882E-09 | 1,33214E-06 | 4,65146E-05 | 0,009603725 | 0,006491309 | 0,000772906 |
| 1 | 1,3567E-11 | 3,8572E-11 | 7,617E-09 | 5,24903E-09 | 8,51841E-10 | 1,56833E-05 | 4,45894E-05 | 0,008805025 | 0,006067888 | 0,00098473 |
| 1 | 8,4652E-12 | | 6,744E-09 | 6,98098E-09 | 7,40171E-10 | 9,56193E-06 | | 0,007618049 | 0,007885444 | 0,000836068 |
| 1 | | 2,4637E-11 | 1,551E-08 | 1,38281E-08 | 7,10375E-10 | | 1,90195E-05 | 0,011976957 | 0,010675284 | 0,000548409 |
| 1 | | | 1,528E-08 | 3,00559E-08 | 2,09422E-09 | | | 0,009881014 | 0,019440576 | 0,001354571 |
| 1 | 5,6417E-12 | 5,5417E-11 | 1,187E-08 | 6,83176E-09 | 6,94549E-10 | 4,65925E-06 | 4,57671E-05 | 0,009807103 | 0,005642117 | 0,000573605 |
| 1 | 3,7396E-12 | | | 6,15523E-09 | 6,48539E-10 | 3,82993E-06 | | | 0,006303937 | 0,000664206 |
| 1 | | 7,3643E-11 | 2,369E-08 | 4,24864E-08 | 1,05516E-09 | | 2,04575E-05 | 0,0065801 | 0,0118025 | 0,00029312 |
| 1 | | 4,8958E-11 | 2,084E-08 | 4,67762E-08 | 8,41589E-10 | | 3,15058E-05 | 0,013409876 | 0,030101971 | 0,00054159 |
| 1 | | | 2,314E-08 | 1,44275E-08 | 1,04066E-09 | | | 0,012423924 | 0,007745799 | 0,000558709 |
| 1 | 1,2624E-11 | 3,2026E-11 | 1,939E-08 | 1,46538E-08 | 1,22826E-09 | 8,79459E-06 | 2,23114E-05 | 0,013510843 | 0,010208714 | 0,000855679 |
| 1 | 6,6732E-12 | 2,5963E-11 | 1,452E-08 | 1,42302E-08 | 7,79745E-10 | 4,99431E-06 | 1,94309E-05 | 0,010866853 | 0,010650142 | 0,000583574 |
| 1 | | | 1,967E-08 | 2,73966E-08 | 4,74179E-10 | | | 0,016994129 | 0,023668535 | 0,000409654 |
| 2 | 4,2421E-12 | | 1,708E-08 | 1,3549E-08 | 5,94933E-10 | 2,45915E-06 | | 0,009904175 | 0,007854447 | 0,000344887 |
| 2 | 1,3932E-11 | | 1,779E-08 | 1,20716E-08 | 9,2183E-10 | 8,04108E-06 | | 0,010269527 | 0,006967361 | 0,000532053 |
| 2 | 1,1983E-11 | 7,9257E-11 | 2,098E-08 | 2,82853E-11 | 9,62125E-10 | 7,22978E-06 | 4,78166E-05 | 0,012657734 | 1,70648E-05 | 0,000580462 |

FIG. 3B

EP 1 799 852 B1

| | | | | | | | | | | |
|---|---|---|---|---|---|---|---|---|---|---|
| 2 | 1,8393E-11 | 4,5662E-11 | 2,015E-08 | | 8,19644E-10 | 1,21817E-05 | 3,02416E-05 | 0,013343311 | | 0,000542849 |
| 2 | | | 1,448E-08 | | 1,39776E-09 | | | 0,009083072 | | 0,000876817 |
| 2 | | 6,836E-11 | 1,449E-08 | | 1,46052E-09 | | 4,77515E-05 | 0,010124602 | | 0,001020214 |
| 2 | | 6,7514E-11 | 1,632E-08 | 1,23761E-08 | 1,43656E-09 | | 4,46469E-05 | 0,010790285 | 0,008184314 | 0,000949995 |
| 2 | 2,1086E-11 | 1,8687E-10 | 4,629E-08 | 4,47053E-08 | 3,36962E-09 | 5,54858E-06 | 4,91725E-05 | 0,012179941 | 0,011763543 | 0,000886667 |
| 2 | | 3,6164E-11 | 1,705E-08 | 2,03369E-08 | 1,04361E-09 | | 3,11724E-05 | 0,014698727 | 0,017530039 | 0,000899572 |
| 2 | 1,4157E-11 | 7,3637E-11 | 2,54E-08 | 1,83929E-08 | 2,52613E-09 | 5,4922E-06 | 2,85671E-05 | 0,009854801 | 0,007135412 | 0,000979997 |
| 2 | 1,883E-11 | 4,6707E-11 | 1,386E-08 | | 1,26756E-09 | 1,31168E-05 | 3,25361E-05 | 0,009651686 | | 0,000882984 |
| 2 | | 4,2657E-11 | 2,274E-08 | 2,22811E-08 | 1,64487E-09 | | 1,81645E-05 | 0,00968381 | 0,009487906 | 0,000700429 |
| 2 | 8,9128E-12 | | 8,142E-09 | 8,80481E-09 | 6,03974E-10 | 9,93859E-06 | | 0,009079291 | 0,00981818 | 0,000673488 |
| 2 | | 5,9026E-11 | 1,017E-08 | 1,03919E-08 | 1,06243E-09 | | 4,87376E-05 | 0,008400077 | 0,0085806 | 0,00087725 |
| 2 | 1,0443E-11 | 3,2098E-11 | 8,908E-09 | | 1,01489E-09 | 8,92106E-06 | 2,742E-05 | 0,007610082 | | 0,000866984 |
| 2 | | | 3,201E-08 | 4,02301E-08 | 1,96169E-09 | | | 0,008282781 | 0,010409207 | 0,000507571 |
| 2 | 1,7362E-11 | 4,6525E-11 | 1,338E-08 | 1,5898E-08 | 8,87509E-10 | 1,08519E-05 | 2,90803E-05 | 0,008366096 | 0,00993708 | 0,00055474 |
| 2 | | 2,1751E-11 | 9,684E-09 | 6,70272E-09 | 1,00721E-09 | | 1,80359E-05 | 0,008029839 | 0,005557926 | 0,000835183 |
| 2 | | 3,9621E-11 | 1,544E-08 | 1,07113E-08 | 7,82337E-10 | | 2,57824E-05 | 0,010045817 | 0,006970055 | 0,000509083 |
| 2 | | 1,7277E-11 | 7,212E-09 | 7,31308E-09 | 5,00092E-10 | | 1,80385E-05 | 0,007530306 | 0,007635398 | 0,000522133 |
| 2 | | | 1,16E-08 | 8,86838E-09 | 4,87726E-10 | | | 0,011343703 | 0,008672349 | 0,000476945 |
| 2 | 6,3045E-11 | 1,1381E-10 | 3,49E-08 | 7,00697E-08 | 2,00379E-09 | 1,4705E-05 | 2,65449E-05 | 0,008139492 | 0,016343587 | 0,000467381 |
| 2 | | 4,7852E-11 | 1,809E-08 | 2,14343E-08 | 1,56275E-09 | | 2,99359E-05 | 0,011319466 | 0,013409263 | 0,000977659 |
| 2 | | 5,3612E-11 | 1,014E-08 | 1,48069E-08 | 1,07675E-09 | | 4,76103E-05 | 0,009001257 | 0,013149386 | 0,000956215 |
| 2 | | 2,4661E-11 | 7,306E-09 | 5,564E-09 | 4,09138E-10 | | 3,12376E-05 | 0,009254274 | 0,007047677 | 0,000518237 |
| 2 | | | 1,001E-08 | 1,0193E-08 | 5,93378E-10 | | | 0,012073071 | 0,012290931 | 0,00071551 |
| 2 | | 2,2834E-11 | 1,273E-08 | 7,08203E-09 | 7,99919E-10 | | 1,97923E-05 | 0,011032633 | 0,006138522 | 0,00069335 |
| 2 | | 1,8917E-11 | 1,203E-08 | 2,6243E-12 | 6,91137E-10 | | 1,99587E-05 | 0,012688462 | 2,76876E-06 | 0,000729181 |
| 2 | | 1,9481E-11 | 8,541E-09 | 7,9433E-09 | 8,65758E-10 | | 2,1838E-05 | 0,009574722 | 0,008904228 | 0,000970492 |
| 2 | | 1,0493E-10 | 2,611E-08 | 1,91831E-08 | 2,04025E-09 | | 3,87354E-05 | 0,009640667 | 0,007081814 | 0,0007532 |
| 2 | | 1,5096E-11 | 1,252E-08 | | 1,03399E-09 | | 2,56194E-05 | 0,02125575 | | 0,001754768 |
| 2 | 1,622E-11 | 2,9618E-11 | 1,77E-08 | 1,11939E-08 | 8,48987E-10 | 1,24237E-05 | 2,26855E-05 | 0,013554529 | 0,008573736 | 0,000650264 |

| PERSON | CORR |
|---|---|
| ANGPT-2/βActin | 0,180937083 |
| OFR7/βActin | 0,273755844 |
| MSRA/βActin | -0,01136342 |
| PPP1R3B/βActin | -0,52459759 |
| NEIL2/βActin | 0,356596 |

FIG. 3C

t-Test: Two-Sample Assuming Equal Variances

ANGPT-2/bActin

|  | inv=0 | inv=2 |
|---|---|---|
| Mean | 6,71378E-06 | 9,24E-06 |
| Variance | 3,72882E-11 | 1,33E-11 |
| Observations | 18 | 12 |
| Pooled Variance | 2,78537E-11 | |
| Hypothesized Mea | 0 | |
| df | 28 | |
| t Stat | -1,285637225 | |
| P(T<=t) one-tail | 0,10455068 | |
| t Critical one-tail | 1,701130259 | |
| P(T<=t) two-tail | 0,20910136 | |
| t Critical two-tail | 2,048409442 | |

t-Test: Two-Sample Assuming Equal Variances
OFR7/bActin

|  | inv=0 | inv=2 |
|---|---|---|
| Mean | 2,44431E-05 | 3,12E-05 |
| Variance | 8,11401E-11 | 1,14E-10 |
| Observations | 19 | 25 |
| Pooled Variance | 9,98402E-11 | |
| Hypothesized Mea | 0 | |
| df | 42 | |
| t Stat | -2,236612098 | |
| P(T<=t) one-tail | 0,015339357 | |
| t Critical one-tail | 1,681951289 | |
| P(T<=t) two-tail | 0,030678715 | |
| t Critical two-tail | 2,018082341 | |

FIG. 3D

t-Test: Two-Sample Assuming Equal Variances
MSRA/bActin

|  | inv=0 | inv=2 |
|---|---|---|
| Mean | 0,010555628 | 0,010577 |
| Variance | 4,81737E-06 | 7,15E-06 |
| Observations | 30 | 32 |
| Pooled Variance | 6,02395E-06 | |
| Hypothesized Mea | 0 | |
| df | 60 | |
| t Stat | -0,034263746 | |
| P(T<=t) one-tail | 0,486390284 | |
| t Critical one-tail | 1,670648544 | |
| P(T<=t) two-tail | 0,972780569 | |
| t Critical two-tail | 2,000297172 | |

t-Test: Two-Sample Assuming Equal Variances
PPP1R3B/bActin

|  | inv=0 | inv=2 |
|---|---|---|
| Mean | 0,017084921 | 0,008825 |
| Variance | 3,73379E-05 | 1,56E-05 |
| Observations | 31 | 26 |
| Pooled Variance | 2,74572E-05 | |
| Hypothesized Mea | 0 | |
| df | 55 | |
| t Stat | 5,927234038 | |
| P(T<=t) one-tail | 1,05415E-07 | |
| t Critical one-tail | 1,673033694 | |
| P(T<=t) two-tail | 2,1083E-07 | |
| t Critical two-tail | 2,004044291 | |

t-Test: Two-Sample Assuming Equal Variances
NEIL2/bActin

|  | inv=0 | inv=2 |
|---|---|---|
| Mean | 0,000548992 | 0,000756 |
| Variance | 3,5877E-08 | 6,94E-08 |
| Observations | 31 | 32 |
| Pooled Variance | 5,28976E-08 | |
| Hypothesized Mea | 0 | |
| df | 61 | |
| t Stat | -3,57938473 | |
| P(T<=t) one-tail | 0,000341293 | |
| t Critical one-tail | 1,670218808 | |
| P(T<=t) two-tail | 0,000682586 | |
| t Critical two-tail | 1,999624146 | |

FIG. 3E

# NEIL2

## Relative expression and C08 inversion status (mean ± SE)

FIG. 4

# BLK

## Relative expression and C08 inversion status (mean ± SE)

FIG. 5

# MSRA

### Relative expression and C08 inversion status (mean ± SE)

FIG. 6

EP 1 799 852 B1

# C8ORF13

## Relative expression and C08 inversion status (mean ± SE)

FIG. 7

# PPP1R3B

### Relative expression and C08 inversion status (mean ± SE)

## FIG. 8

# TNKS

**Relative expression and C08 inversion status (mean ± SE)**

FIG. 9

# CTSB

## Relative expression and C08 inversion status (mean ± SE)

FIG. 10

EP 1 799 852 B1

ANGPT

Relative expression and C08 inversion status (mean ± SE)

FIG. 11

# TDH
## Relative expression and C08 inversion status (mean ± SE)

FIG. 12

EP 1 799 852 B1

# C8ORF7

## Relative expression and C08 inversion status (mean ± SE)

FIG. 13

EP 1 799 852 B1

-> BLK

|  | Value | Std. Error | t value | Pr(>\|t\|) |
|---|---|---|---|---|
| (Intercept) | -4.5501 | 0.0734 | -62.0103 | 0.0000 |
| SLOPE INV CNT | -0.0413 | 0.0559 | -0.7380 | 0.4624 |

Residual standard error: 0.4366 on 91 degrees of freedom

R-Squared: 0.00595

-> MSRA

|  | Value | Std. Error | t value | Pr(>\|t\|) |
|---|---|---|---|---|
| (Intercept) | -4.6124 | 0.0389 | -118.4654 | 0.0000 |
| SLOPE INV CNT | 0.0101 | 0.0296 | 0.3407 | 0.7341 |

Residual standard error: 0.2311 on 90 degrees of freedom

R-Squared: 0.001288

-> C8ORF13

|  | Value | Std. Error | t value | Pr(>\|t\|) |
|---|---|---|---|---|
| (Intercept) | -8.0482 | 0.0951 | -84.5920 | 0.0000 |
| SLOPE INV CNT | 0.4407 | 0.0725 | 6.0761 | 0.0000 |

Residual standard error: 0.5661 on 91 degrees of freedom

R-Squared: 0.2886

-> NEIL2

|  | Value | Std. Error | t value | Pr(>\|t\|) |
|---|---|---|---|---|
| (Intercept) | -7.5209 | 0.0559 | -134.4702 | 0.0000 |
| SLOPE INV CNT | 0.1556 | 0.0426 | 3.6488 | 0.0004 |

Residual standard error: 0.3328 on 91 degrees of freedom

R-Squared: 0.1276

FIG. 14A

-> PPP1R3B

| | Value | Std. Error | t value | Pr(>\|t\|) |
|---|---|---|---|---|
| (Intercept) | -4.2104 | 0.0670 | -62.8642 | 0.0000 |
| SLOPE INV CNT | -0.2871 | 0.0546 | -5.2605 | 0.0000 |

Residual standard error: 0.3962 on 83 degrees of freedom
R-Squared: 0.25

-> TNKS

| | Value | Std. Error | t value | Pr(>\|t\|) |
|---|---|---|---|---|
| (Intercept) | 0.0134 | 0.0003 | 48.7031 | 0.0000 |
| SLOPE INV CNT | -0.0121 | 0.0064 | -1.8806 | 0.0635 |

Residual standard error: 0.002235 on 83 degrees of freedom
R-Squared: 0.04087

-> CTSB

| | Value | Std. Error | t value | Pr(>\|t\|) |
|---|---|---|---|---|
| (Intercept) | -2.0358 | 0.0289 | -70.3332 | 0.0000 |
| SLOPE INV CNT | -0.0727 | 0.0220 | -3.3010 | 0.0014 |

Residual standard error: 0.1718 on 90 degrees of freedom
R-Squared: 0.108

-> ANGPT2

| | Value | Std. Error | t value | Pr(>\|t\|) |
|---|---|---|---|---|
| (Intercept) | -12.1536 | 0.1194 | -101.7581 | 0.0000 |
| SLOPE INV CNT | 0.0745 | 0.0911 | 0.8180 | 0.4160 |

Residual standard error: 0.6436 on 73 degrees of freedom
R-Squared: 0.009083

FIG. 14B

-> TDH (not log-transformed relative expression)

|  | Value | Std. Error | t value | Pr(>\|t\|) |
|---|---|---|---|---|
| (Intercept) | 0.0000 | 0.0000 | 17.4644 | 0.0000 |
| SLOPE INV CNT | 0.0000 | 0.0000 | -3.7742 | 0.0003 |

Residual standard error: 1.404e-05 on 75 degrees of freedom
R-Squared: 0.1596

-> C8ORF7

|  | Value | Std. Error | t value | Pr(>\|t\|) |
|---|---|---|---|---|
| (Intercept) | -10.6607 | 0.0683 | -156.1230 | 0.0000 |
| SLOPE INV CNT | 0.1187 | 0.0518 | 2.2908 | 0.0245 |

Residual standard error: 0.3799 on 82 degrees of freedom
R-Squared: 0.06015

FIG. 14C

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0005407 A
- WO 05040427 A2

- WO 05040427A2 A

**Non-patent literature cited in the description**

- The global burden of disease: a comprehensive assessment of mortality and disability from diseases, injuries, and risk factors in 1990 and projected to 2020. Harvard University Press, 1996
- **Puig, M. et al.** *Proc. Natl. Acad. Sci. USA,* 2004, vol. 101, 9013-8
- **Schaeffer, S. et al.** *Proc. Natl. Acad. Sci. USA,* 2003, vol. 100, 8319-24

- **Charlesworth, B.** *Genet Res.,* 1974, vol. 23, 259-80
- **Giglio et al.** *Am. J. Hum. Genet.,* vol. 68, 874-83
- *User Bulletin #2, ABI Prism 7700 Sequence Detection System,* 11 December 1997